(19) Europäisches Patentamt
European Patent Office
Office européen des brevets

(11) Publication number : **0 481 802 A1**

(12) # EUROPEAN PATENT APPLICATION

(21) Application number : **91309618.6**

(22) Date of filing : **17.10.91**

(51) Int. Cl.[5] : **C07D 413/12,** C07D 413/06, C07D 213/64, C07D 213/70, C07D 213/73, A61K 31/44, // (C07D413/12, 263:00, 213:00), (C07D413/06, 263:00, 213:00)

The applicant has filed a statement in accordance with Rule 28 (4) EPC (issue of a sample only to an expert). Accession number(s) of the deposit(s): ATCC 53771.

(30) Priority : **18.10.90 US 599968**
**07.06.91 US 712326**
**07.10.91 US 769580**

(43) Date of publication of application :
**22.04.92 Bulletin 92/17**

(84) Designated Contracting States :
**AT BE CH DE DK ES FR GB GR IT LI LU NL SE**

(71) Applicant : **MERCK & CO. INC.**
**126, East Lincoln Avenue P.O. Box 2000**
**Rahway New Jersey 07065-0900 (US)**

(72) Inventor : **Chen, Shieh-Shung Tom**
**12 Scott Drive**
**Morganville, NJ 07751 (US)**
Inventor : **Hsu, Annjia**
**4180 Randmore Court**
**Columbus, Ohio 43220 (US)**
Inventor : **Doss, George A.**
**39 Normany Drive**
**Westfield, NJ 07090 (US)**
Inventor : **Goldman, Mark E.**
**1510 Bridal Path Road**
**Lansdale, PA 19446 (US)**
Inventor : **Balani, Suresh K.**
**Danbeigh Drive**
**Hatfield, PA 19440 (US)**
Inventor : **Theoharides, Anthony D.**
**417 Shipwrighter Way**
**Lansdale, PA 19446 (US)**
Inventor : **Hoffman, Jacob M., Jr.**
**Pennbrooke Gardens, Apt. 17A**
**North Wales, PA 19454 (US)**
Inventor : **Pitzenberger, Steven M.**
**901 Perkiomen Avenue**
**Lansdale, PA 19446 (US)**
Inventor : **Ramjit, Harri G.**
**313 Cowpath Road**
**Lansdale, PA 19446 (US)**
Inventor : **Kauffman, Laura R.**
**535 Canterbury Road**
**Jeffersonville, PA 19403 (US)**
Inventor : **Arison, Byron H.**
**88 Century Lane**
**Watchung, NJ 07060 (US)**
Inventor : **Saari, Walfred S.**
**1740 Wagon Wheel Lane**
**Lansdale, PA 19446 (US)**
Inventor : **Rooney, Clarence S.**
**2902 Hickory Hill Drive**
**Worcester, PA 19490 (US)**
Inventor : **Wai, John S.**
**208 Tanglewood Way**
**Harleysville, PA 19438 (US)**

(74) Representative : **Hesketh, Alan, Dr. et al**
**European Patent Department Merck & Co., Inc. Terlings Park Eastwick Road**
**Harlow Essex, CM20 2QR (GB)**

(54) **Hydroxylated inhibitors of HIV reverse transcriptase.**

(57) Novel biotransformed or synthetic hydroxy pyridinones inhibit HIV reverse transcriptase, and are useful in the prevention or treatment of infection by HIV and the treatment of AIDS, either as compounds, pharmaceutically acceptable salts (where appropriate), pharmaceutical composition ingredients, whether or not in combination with other antivirals, anti-infectives, immunomodulators, antibiotics or vaccines. Methods of treating AIDS and methods of preventing or treating infection by HIV are also described.

EP 0 481 802 A1

The present invention is concerned with compounds which inhibit the reverse transcriptase encoded by human immunodeficiency virus (HIV). The compounds are of value in the prevention of infection by HIV, the treatment of infection by HIV and the treatment of the resulting acquired immune deficiency syndrome (AIDS). The present invention also relates to pharmaceutical compositions containing the compounds and to a method of use of the present compounds with or without other agents for the treatment of AIDS & viral infection by HIV.

## BACKGROUND OF THE INVENTION

A retrovirus designated human immunodeficiency virus (HIV) is the etiological agent of the complex disease that includes progressive destruction of the immune system (acquired immune deficiency syndrome; AIDS) and degeneration of the central and peripheral nervous system. This virus was previously known as LAV, HTLV-III, or ARV. A common feature of retrovirus replication is reverse transcription of the RNA genome by a virally encoded reverse transcriptase to generate DNA copies of HIV sequences, a required step in viral replication. It is known that some compounds are reverse transcriptase inhibitors and are effective agents in the treatment of AIDS and similar diseases, e.g., azidothymidine or AZT.

Nucleotide sequencing of HIV shows the presence of a pol gene in one open reading frame [Ratner, L. et al., Nature, 313, 277(1985)]. Amino acid sequence homology provides evidence that the pol sequence encodes reverse transcriptase, an endonuclease and an HIV protease [Toh, H. et al., EMBO J. 4, 1267 (1985); power, M.D. et al., Science, 231, 1567 (1986); Pearl, L.H. et al., Nature 329., 351 (1987)].

Applicants demonstrate that the compounds of this invention are inhibitors of HIV reverse transcriptase. The inhibition is very specific, since there is little or no inhibition of the reverse transcriptases of AMV, MMLV or SIV. Further, the compounds of the present invention do not require bio-activation to be effective. They are also generally more potent than AZT.

Applicants have discovered that hydroxylation in certain positions in the pyridinone ring confer the simultaneous advantages of compounds that are both metabolic products and potent inhibitors of HIV reverse transcriptase. Some of the lead compounds of the present invention are biotransformations of an HIV reverse transcriptase inhibitor L-696,040. The biotransforming microorganism is Actinoplanacete sp. (Merck Culture Collection MA 6559) ATCC No. 53771. Applicants have now also synthesized a variety of analogs.

## BRIEF DESCRIPTION OF THE INVENTION

Compounds as herein defined are disclosed. These compounds are useful in the inhibition of HIV reverse transcriptase, the prevention of infection by HIV, the treatment of infection by HIV and in the treatment of AIDS and/or ARC, either as compounds, pharmaceutically acceptable salts (when appropriate), pharmaceutical composition ingredients, whether or not in combination with other antivirals, anti-infectives, immunomodulators, antibiotics or vaccines. Methods of treating AIDS, methods of preventing infection by HIV, and methods of treating infection by HIV are also disclosed.

## DETAILED DESCRIPTION OF THE INVENTION AND PREFERRED EMBODIMENTS

This invention is concerned with the compounds of formula I, combinations thereof, or pharmaceutically acceptable salts thereof, in the inhibition of HIV reverse transcriptase, the prevention or treatment of infection by HIV and in the treatment of the resulting acquired immune deficiency syndrome (AIDS). Compounds of the present invention are compounds of Formula I, which includes biotransformed products of an HIV reverse transcriptase inhibitor, L-696,040. Compounds of formula I have the following structure:

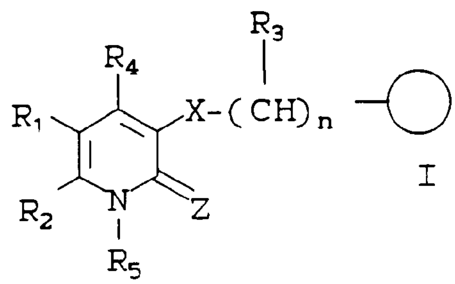

I

wherein

$R_1$ or $R_2$ or both are substituted at least once with OH;

X is 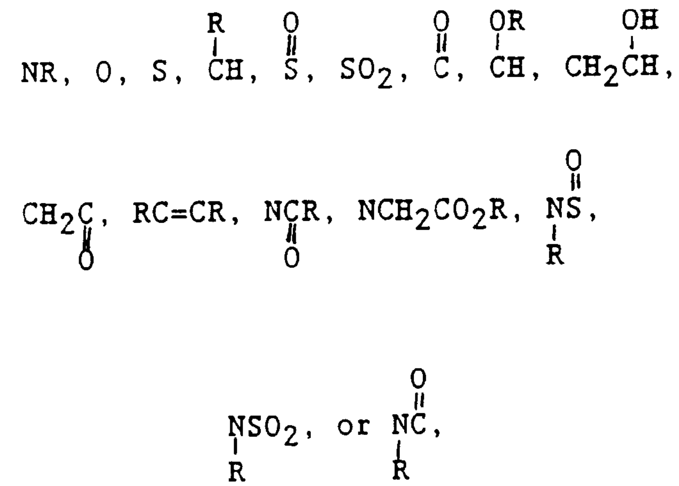

where R is H, $C_{1-8}$ alkyl, $C_{1-8}$ alkenyl or $C_{3-8}$ cycloalkyl;
Z is O, S or $NR_x$ when $R_x$ is H or $C_{1-8}$ alkyl;
n is 0-4;
$R_1$, $R_2$ and $R_4$ are the same or different and are independently

(i) H;
(ii) $C_{1-8}$ alkyl, $C_{1-8}$ alkenyl, $C_{3-8}$cycloalkyl; any of which is unsubstituted or substituted with one or two of $C_{1-3}$ alkoxy, $C_{1-4}$ alkylamino, di($C_{1-4}$ alkyl)amino, $C_{1-3}$ alkylthio, hydroxy, amino, oxo, carbonyl, aminocarbonyl, or oximido, or one to five of halo;
(iii) $C_{1-5}$ alkylthio;
(iv) $C_{1-5}$ alkylsulfinyl;
(v) $C_{1-5}$ alkylsulfonyl;
(vi) $C_{1-5}$ alkoxy;
(vii) $C_{1-5}$ alkoxycarbonyl;
(viii) cyano; or
(ix) aryl;

or, $R_1$ and $R_4$ may together form a cycloalkyl ring containing 5-7 members;
or, $R_1$ and $R_2$ may together form a cycloalkyl ring containing 5-7 members;
and $R_3$ or $R_5$ are the same or different and are independently

(i) H;
(ii) $C_{1-8}$ alkyl;
(iii) $C_{1-8}$ alkenyl;
(iv) $C_{3-8}$ cycloalkyl;

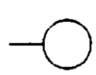

is aryl or heterocycle, each unsubstituted or substituted with one or more of

(i) $C_{1-6}$ alkyl unsubstituted or substituted with one or more of A, wherein A is halo, hydroxy, hydroxy-$C_{1-4}$ alkyl, amino, $C_{1-6}$ alkylamino, di($C_{1-6}$ alkyl)amino or aryl;
(ii) $C_{1-6}$ alkenyl unsubstituted or substituted with one or more of A;
(iii) $C_{3-6}$ cycloalkyl unsubstituted or substituted with one or more of A;
(iv) $C_{1-6}$ alkoxy unsubstituted or substituted with one or more of A;
(v) aryl;
(vi) amino;
(vii) $C_{1-6}$ alkylamino;

(viii) di($C_{1-6}$ alkyl)amino;
(ix) amino-$C_{1-8}$ alkyl;
(x) $C_{1-8}$ alkyl-amino-$C_{1-8}$ alkyl;
(xi) di-($C_{1-6}$ alkyl)amino $C_{1-8}$ alkyl;
(xii) $C_{1-6}$ alkoxycarbonyl;
(xiii) aminocarbonyl;
(xiv) $C_{1-6}$ alkyl aminocarbonyl;
(xv) di($C_{1-6}$ alkyl)aminocarbonyl;
(xvi) $C_{1-6}$ alkylthio;
(xvii) $C_{1-6}$ alkylsulfinyl;
(xviii) $C_{1-6}$ alkylsulfonyl;
(xix) hydroxy;
(xx) halo;
(xxi) CN; or
(xxii) $NO_2$;

with the proviso that heterocycle is not phthalimide;
or pharmaceutically acceptable salt or ester thereof.

A first embodiment is a compound of formula II,

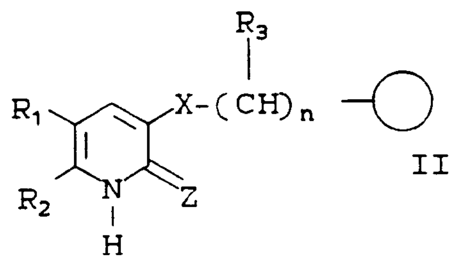

II

wherein:
X is NH or $CH_2$;
Z is O or S;
n is 1-4;
$R_1$ is
(i) $C_{1-8}$ alkyl, unsubstituted or substituted with one or two of $C_{1-3}$alkoxy, halo, oxo, hydroxyl, $C_{1-4}$alkylamino, $C_{1-4}$-di-alkylamino, or $C_{1-3}$ alkylthio;
(ii) $C_{1-3}$ alkylthio;
(iii) $C_{1-3}$ alkoxy; or
$R_2$ is H, methyl or ethyl, unsubstituted or substituted with one or two of methoxy, hydroxyl, methylamino, dimethylamino or methylthio;
provided that one of $R_1$ or $R_2$, or both, is substituted at least once with hydroxyl;
$R_3$ is H or $C_{1-8}$ alkyl;

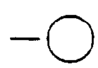

is aryl or heterocycle, each unsubstituted or substituted with one or more of
(a) $C_{1-6}$ alkyl,
(b) $C_{1-6}$ alkoxy unsubstituted or substituted with hydroxy-$C_{1-4}$ alkyl,
(c) amino,
(d) $C_{1-6}$ alkyl amino,
(e) di($C_{1-6}$ alkyl)amino,
(f) amino- $C_{1-8}$ alkyl

(g) $C_{1-8}$ alkyl-amino-$C_{1-8}$ alkyl,
(h) di-($C_{1-6}$ alkyl)amino $C_{1-8}$ alkyl,
(i) hydroxy, or
(j) halo,

with the proviso that heterocycle is not phthalimide, or pharmaceutically acceptable salt or ester thereof.

A second embodiment is further limited to a compound of the formula:

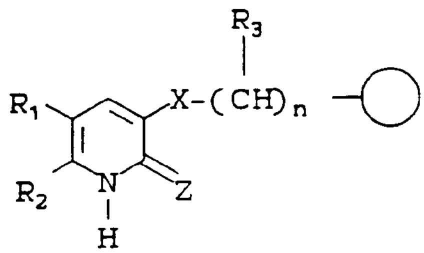

wherein:
X is NH;
Z is O or S;
n is 1-4;
$R_1$ is
(i) $C_{1-8}$ alkyl, unsubstituted or substituted with one or two of $C_{1-3}$alkoxy, halo, hydroxyl, $C_{1-4}$alkylamino, $C_{1-4}$-di-alkylamino, or $C_{1-3}$ alkylthio;
(ii) $C_{1-3}$ alkylthio;
(iii) $C_{1-3}$ alkoxy; or
( iv) halo;
$R_2$ is H, methyl or ethyl, unsubstituted or substituted with one or two of methoxy, hydroxyl, methylamino, dimethylamino or methylthio;
provided that one of $R_1$ or $R_2$, or both, is substituted at least once with hydroxyl;
$R_3$ is H or $C_{1-8}$ alkyl;

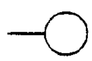

is aryl or heterocycle, each unsubstituted or substituted with one or more of
(a) $C_{1-6}$ alkyl,
(b) $C_{1-6}$ alkoxy unsubstituted or substituted with hydroxy-$C_{1-4}$ alkyl,
(c) amino,
(d) $C_{1-6}$ alkyl amino,
(e) di($C_{1-6}$ alkyl)amino,
(f) amino- $C_{1-8}$ alkyl,
(g) $C_{1-8}$ alkyl-amino-$C_{1-8}$ alkyl,
(h) di-($C_{1-6}$ alkyl)amino $C_{1-8}$ alkyl,
(i) hydroxyl, or
(j) halogen,

with the proviso that heterocycle is not phthalimide, or pharmaceutically acceptable salt or ester thereof.

A third embodiment is further limited to a compound wherein
$R_1$ is $C_{1-4}$ alkyl, unsubstituted or substituted with one or more of hydroxyl;
$R_2$ is H, $CH_3$ or $CH_2OH$;
provided that one or $R_1$ or $R_2$, or both, is substituted at least once with hydroxyl;
aryl is
(a) naphthyl, unsubstituted or substituted with one or more of $C_{1-6}$ alkyl, $C_{1-6}$ alkoxy, halogen or hydroxyl;
(b) phenyl, unsubstituted or substituted with one or more of $C_{1-6}$ alkyl, $C_{1-6}$ alkoxy, halogen or hydroxyl; or

(c) biphenyl.

A fourth embodiment is a compound of the first embodiment wherein heterocycle is thienyl, oxazolyl, thiazolyl, triazolyl, pyridyl unsubstituted or substituted with one or more of $C_{1-2}$alkoxy or $C_{1-3}$alkyl or hydroxy alkyl, pyridinonyl, pyrazinyl, benzothienyl, quinolinyl unsubstituted or substituted with $C_{1-2}$ alkoxy, 5,6,7,8-tetrahydroquinolinyl unsubstituted or substituted with $C_{1-2}$ alkoxy, isoquinolinyl unsubstituted or substituted with $C_{1-2}$ alkoxy, benzimidazolyl, benzofuranyl, benzothiazolyl, benzoxazolyl unsubstituted or substituted with one or more of $C_{1-6}$ alkyl or halogen or hydroxyl, 4,5,6,7-tetrahydrobenzoxazolyl, naphth [3,2-d] oxazolyl, oxazolo [4,5-b] pyridyl, chromone, or benzopyrimidinone.

A fifth embodiment is further limited to a compound wherein heterocycle is 2-oxazolyl, 2-pyridyl, 3-pyridyl unsubstituted or substituted with one or more of $C_{1-2}$alkoxy or $C_{1-3}$alkyl, 2-benzothienyl, 2-quinolinyl, 2-(5,6,7,8-tetrahydroquinolinyl) unsubstituted or substituted with $C_{1-2}$ alkoxy, 2-benzimidazolyl, 2-benzofuranyl, 2-benzothiazolyl, 2-oxazolo [4,5-b] pyridyl, or 2-benzoxazolyl unsubstituted or substituted with one or more of methyl, halogen or hydroxyl.

A sixth embodiment is further limited to a compound wherein heterocycle is 2-methoxy-5,6-dimethyl-3-pyridyl, 2-methoxy-5-ethyl-6-methyl-3-pyridyl, 3-methoxy-5,6-dimethyl-2-pyridyl, 3-methoxy-5-methyl-6-ethyl-2-pyridyl, 2-benzoxazolyl, 7-methyl-2-benzoxazolyl, 4,7-dimethyl-2-benzoxazolyl, 7-chloro-2-benzoxazolyl, 4,7-dichloro-2-benzoxazolyl, 4-fluoro-2-benzoxazolyl, or 4,7-difluoro-2-benzoxazolyl.

A seventh embodiment is further limited to a compound wherein Heterocycle is 2-methoxy-5-ethyl-6-methyl-3-pyridyl, 2-benzoxazolyl, 7-methyl-2-benzoxazolyl, 4,7-dimethyl-2-benzoxazolyl, 7-chloro-2-benzoxazolyl, or 4,7-dichloro-2-benzoxazolyl.

The most preferred compounds include the following:

A.

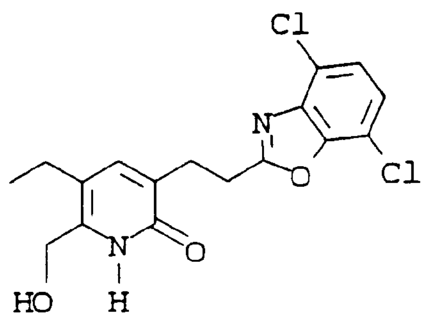

3-[2-(4,7-dichlorobenzoxazol-2-yl)ethyl]-5-ethyl-6-hydroxymethylpyridin-2(1H)-one;

B.

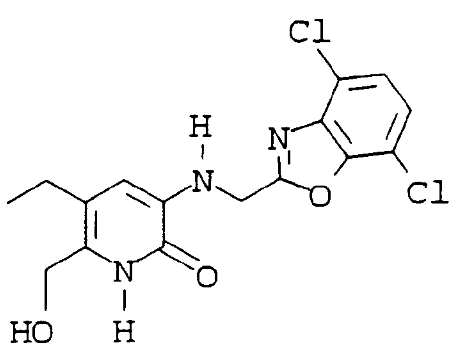

3-[(4,7-dichlorobenzoxazol-2-yl)methylamino]-5-ethyl-6-hydroxymethylpyridin-2(1H)-one, or

C.

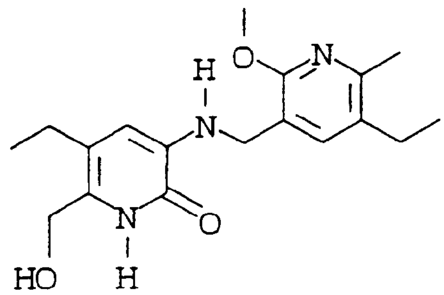

3-{N-[(2-methoxy-5-ethyl-6-methylpyridin-3-yl)methyl]amino}-5-ethyl-6-hydroxymethylpyridin-2(1H)-one, or pharmaceutically acceptable ester thereof.

Other preferred compounds are synthesized by ATCC 53771 from precursor L-696,040, and have the structure:

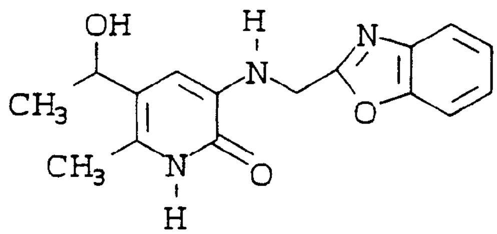

L-697, 913

or

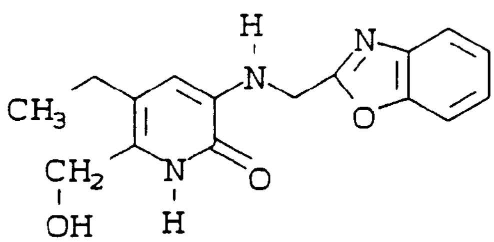

L-697, 914

or pharmaceutically acceptable ester thereof.

The biotransforming microoganism is Actinoplanacete sp. (Merck Culture Collection MA 6559), deposited at the American Type Culture Collection (ATCC 53771).

The compounds of this invention also comprise the following:

3-[(benzoxazol-2-yl)methylamino]-5-(1-hydroxyethyl)-6-methylpyridin-2(1H)-one,
3-[(benzoxazol-2-yl)methylamino]-5-ethyl-6-hydroxymethylpyridin-2(1H)-one,
3-[2-(benzoxazol-2-yl)ethyl]-5-ethyl-6-hydroxymethylpyridin-2(1H)-one,
3-[(4,7-dimethylbenzoxazol-2-yl)methylamino]-5-ethyl-6-hydroxymethylpyridin-2(1H)-one,
3-[2-(benzoxazol-2-yl)ethyl]-5-(1-hydroxyethyl)-6-methylpyridin-2(1H)-one,
3-[(4,7-dimethylbenzoxazol-2-yl)methylamino]-5-(1-hydroxyethyl)-6-methylpyridin-2(1H)-one,
3-[2-(4,7-dichlorobenzoxazol-2-yl)ethyl]-5-ethyl-6-hydroxymethylpyridin-2(H)-one,
3-{N-[(2-methoxy-5-ethyl-6-methylpyridin-3-yl)methyl]amino}-5-ethyl-6-hydroxymethylpyridin-2(1H)-one,
3-[(4,7-dichlorobenzoxazol-2-yl)methylamino]-5-ethyl-6-hydroxymethylpyridin-2(1H)-one,

3-[2-(2-methoxy-5-ethyl-6-methylpyridin-3-yl)ethyl]-5-ethyl-6-hydroxymethylpyridin-2(1H)-one,
3-[(4,7-dichlorobenzoxazol-2-yl)methylamino]-5-(1-hydroxyethyl)-6-methylpyridin-2(1H)-one,
3-{N-[(2-methoxy-5-ethyl-6-methylpyridin-3-yl)methyl]amino}-5-(1-hydroxyethyl)-6-methylpyridin-2(1H)-one,
3-[2-(4,7-Dichlorobenzoxazol-2-yl)ethyl]-5-ethyl-6-hydroxymethylpyridin-2(1H)-thione,
3-[(4,7-Dichlorobenzoxazol-2-yl)methyl]amino-5-[1(R,S)-hydroxyethyl]-6-methylpyridin-2(1H)-one,
3-[(4,7-Dichlorobenzoxazol-2-yl)methyl]amino-5-(1-oxoethyl)-6-methylpyridin-2(1H)-one,
3-[(4,7-dichlorobenzoxazol-2-yl)methyl]amino-5-[1(S,R)-hydroxyethyl)-6-methylpyridin-2(1H)-one,
3-[(5-Ethyl-2-methoxy-6-methylpyridin-3-yl)methyl]amino-5-[1(R,S)-hydroxyethyl]-6-methylpyridin-2(1H)-one,
5-Ethyl-3-[2-(4-fluorobenzoxazol-2-yl)ethyl]-6-hydroxymethylpyridin-2(1H)-one,
3-{N-[(5,6-Dimethyl-3-methoxypyridin-2-yl)methyl]amino}-5-ethyl-6-hydroxymethylpyridin-2(1H)-one,
5-Ethyl-6-hydroxymethyl-3-{N-[(3-methoxy-5,6,7,8-tetrahydroquinolin-2-yl)methyl]amino}pyridin-2(1H)-one,
5-Ethyl-3-{N-[(6-ethyl-3-methoxy-5-methylpyridin-2-yl)methyl]amino}-6-hydroxymethylpyridin-2(1H)-one,
3-{N-[(5,6-dimethyl-2-methoxypyridin-3-yl)methyl]-amino}-5-ethyl-6-hydroxymethylpyridin-2(1H)-one,
5-ethyl-6-hydroxymethyl-3-{N-[(2-methoxy-5,6,7,8-tetrahydroquinolin-3-yl)methyl]amino}pyridin-2-(1H)-one,
3-{N-[(2-methoxy-5,6-trimethylene-pyridin-3-yl)methyl]amino}-5-ethyl-6-hydroxymethylpyridin-2-(1H)-one,
3-{N-[(6-methoxy-indan-5-yl)methyl]amino}-5-ethyl-6-hydroxymethylpyridin-2(1H)-one,
3-{N-[(5-ethyl-2-methoxypyridin-3-yl)methyl]amino}-5-ethyl-6-hydroxymethylpyridin-2(1H)-one,
5-ethyl-6-hydroxymethyl-3-{N-[(2-methoxyquinolin-3-yl)methyl]amino}pyridin-2(1H)-one,
3-{N-[(3-methoxy-5,6-trimethylene-pyridin-2-yl)-methyl]amino}-5-ethyl-6-hydroxymethylpyridin-2(1H)-one,
3-{N-[(3-methoxy-5-ethylpyridin-2-yl)methyl]amino}-5-ethyl-6-hydroxymethylpyridin-2(1H)-one,
3-[N-(5-ethyl-2-methoxybenzyl)amino]-5-ethyl-6-hydroxymethylpyridin-2(1H)-one,
3-[2-(4,7-difluorobenzoxazol-2-yl)ethyl]-5-ethyl-6-hydroxmethylpyridin-2(1H)-one,
3-[N-(4,5-dimethyl-2-methoxybenzyl)amino]-5-ethyl-6-hydroxymethylpyridin-2(1H)-one,
or pharmaceutically acceptable salt or ester thereof.

The compounds of the present invention, may have asymmetric centers and occur as racemates, racemic mixtures and as individual diastereomers, with all isomeric forms being included in the present invention. Also, combinations of substituents and/or variables are permissible only if such combinations result in stable compounds.

When any variable (e.g., aryl, heterocycle, $R_1$, $R_2$, $R_3$, etc.) occurs more than one time in any constituent or in formula I, its definition on each occurrence is independent of its definition at every other occurrence. Also, combinations of substituents and/or variables are permissible only if such combinations result in stable compounds.

As used herein except where noted, "alkyl" is intended to include both branched- and straightchain saturated aliphatic hydrocarbon groups having the specified number of carbon atoms; "alkoxy" represents an alkyl group of indicated number of carbon atoms attached through an oxygen bridge. "Halogen" or "halo" as used herein, means fluoro, chloro, bromo and iodo.

As used herein, with exceptions as noted, "aryl" is intended to mean any stable monocyclic, bicyclic or tricyclic carbon ring of up to 7 members in each ring, wherein at least one ring is aromatic. Examples of such aryl elements include phenyl, naphthyl, tetrahydronaphthyl, biphenyl, phenanthryl, anthryl or acenaphthyl.

The term heterocycle or heterocyclic, as used herein except where noted, represents a stable 5- to 7-membered monocyclic or stable 8- to 11-membered bicyclic or stable 11-15-membered tricyclic heterocyclic ring which is either saturated or unsaturated, and which consists of carbon atoms and from one to four heteroatoms selected from the group consisting of N, O and S, and wherein the nitrogen and sulfur heteroatoms may optionally be oxidized, and the nitrogen heteroatom may optionally be quaternized, and including any bicyclic group in which any of the above-defined heterocyclic rings is fused to a benzene ring. The heterocyclic ring may be attached at any heteroatom or carbon atom which results in the creation of a stable structure. Examples of such heterocyclic elements include thienyl, oxazolyl, thiazolyl, triazolyl, pyridyl, pyrazinyl, benzothienyl, quinolinyl, isoquinolinyl, benzimidazolyl, benzofuranyl, benzothiazolyl, benzoxazolyl unsubstituted or substituted with one or more of $C_{1-6}$ alkyl or halogen or hydroxyl, naphth [3,2-d] oxazolyl, oxazolo [4,5-b] pyridyl, chromone, or benzopyrimidinone. Heterocycle does not include phthalimide.

The compounds of the present inventions are useful in the inhibition of HIV reverse transcriptase, the prevention or treatment of infection by the human immunodeficiency virus (HIV) and the treatment of consequent pathological conditions such as AIDS. Treating AIDS or preventing or treating infection by HIV is defined as including, but not limited to, treating a wide range of states of HIV infection: AIDS, ARC (AIDS related complex), both symptomatic and asymptomatic, and actual or potential exposure to HIV. For example, the compounds of this invention are useful in treating infection by HIV after suspected past exposure to HIV by e.g., blood transfusion, organ transplant, exchange of body fluids, bites, accidental needle stick, or exposure to patient blood

during surgery.

In the organic synthesis of the amino pyridones (3) of this invention, the procedures and protocols of U.S. 3,721,676 can be followed for making many intermediates, which patent is incorporated by reference for these purposes. Applicants here provide preferred methods of synthesis, outlined below.

The 3-nitropyridone of the first step in the synthesis of 3 may be formed by a simultaneous condensation and cyclization of

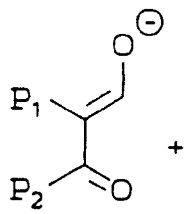

+

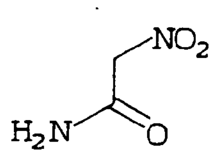

in the presence of a base such as piperidinium acetate. Nitroacetamide is prepared according to Brownstein, S.K., J. Org. Chem. 23, 113 (1958).

When the side chains $R_1$ and/or $R_2$ are hydroxylated, they may have an OH-protecting group (P) such as 2-benzyloxy. $P_1$ and $P_2$ are thus defined as:

$P_1$ is $R_1$- or P-$R_1$-;

$P_2$ is $R_2$- or P-$R_2$-.

Synthesis of 3 with a hydroxylation on a side chain at the 5-position or 6-position of the amino pyridone ring can be carried out with compounds wherein $R_1$ or $R_2$ are protected.

The 3-nitropyridone products of the first step can also be prepared by direct nitration of 3-unsubstituted pyridones. The 3-nitropyridone product of the first step

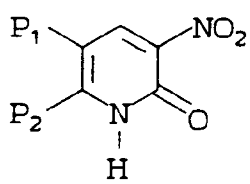

1

is reduced to

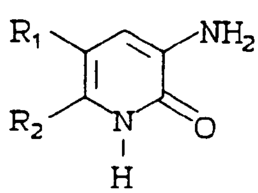

2

in a second step, preferably by catalytic reduction (when sulfur atoms are not present) in the presence of e.g. $H_2$ gas and Pd on carbon catalyst in a solvent such as ethanol. See e.g., Okafor, C.O. et al., J. Heterocyclic Chem. 20, 199 (1983). Alternatively the reduction of the second step (including when sulfur atoms are present) can be performed by chemical means, e.g. with NaSH, $Na_2S_2O_4$, Fe + $CaCl_2$, $H_2S$, or Sn + HCl. Reduction with iron in the presence of calcium chlorides is described in Okafor, C.O., J. Org. Chem. 47, 592 (1982). A third step, the final one in the process giving rise to the compounds of this invention,

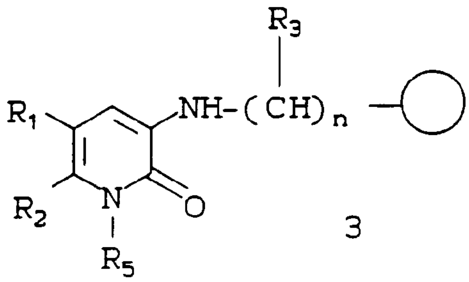

3

involves a coupling reaction, either by alkylation with an alkylhalide or a reductive alkylation with an aldehyde.

Additional reduction by hydrogenation over Pd on carbon can be performed to remove any remaining OH-protecting groups, as needed.

Carba derivatives (9), having alkylene bridges between the pyridone ring and the heterocyclic or aromaticentity attached at the 3-position, may be made by the following methods, the first being the preferred method.

In a first step 3-cyano-2-(1H) pyridinone is prepared by a simultaneous condensation and cyclization of

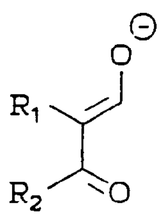

+

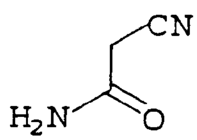

in the presence of base such as piperidinium acetate or pyrrolidine acetate. The resulting 3-cyano-2-(1H) pyridone,

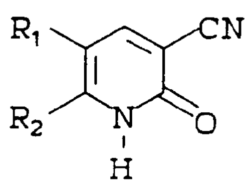

, 4

is heated in the presence of phosphorus pentachloride and/or $POCl_3$ to form the corresponding chloro pyridine,

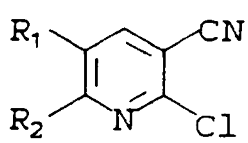

, 5

a conventional method of converting pyridones to chloropyridines. $PBr_3$ is useful for making the corresponding bromine derivative of product 5. Product 5 is then subjected to nucleophilic substitution to attach an alkoxy protecting group:

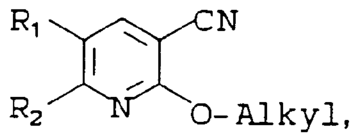

6

Reduction in the presence of, for example, diisobutyl aluminum hydride, yields

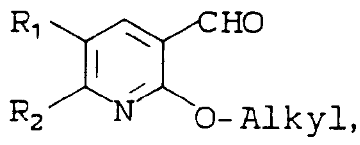

7

which is a conventional method of reducing nitriles to aldehydes. An alternative reducing agent for the synthesis of 7 is lithium trialkoxy aluminum hydride.

Condensation in the presence of base at very low temperature (preferably at least -100°C) with an alkyl-substituted aryl or alkyl substituted heterocycle yields

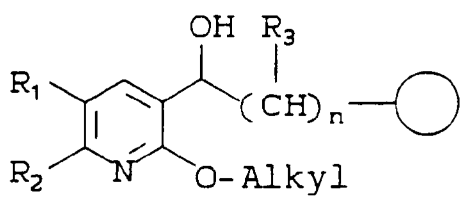

8

(n = 1)

Conditions will vary with the heterocycle to be attached. (The Wittig approach, infra, is preferable when n is more than 1.) Dehydration of the alcohol, and dealkylation followed by hydrogenation results in the compounds of the invention, wherein X = $CH_2$,

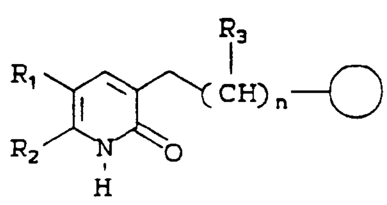

9

(n = 1)

The dealkylation is typically carried out with pyridine hydrochloride in the presence of heat. The dehydration need not occur in the same reaction, but may be performed separately by conventional means. The standard catalyst for hydrogenation to produce product 9 is palladium, but other noble metals may substitute. Alternatively, the hydrogenation can be carried out with diimide or Raney nickel. $BCl_3$ or $BBr_3$ at low temperature is useful for performing dealkylation without dehydration. Dealkylation can also be carried out with KI in glacial acetic acid.

A second alternative variation on the final 3 steps is the Wittig approach, wherein the aldehyde derivative

(product 7 above) is reacted with aryl or heterocycle, each substituted with alkylene triphenylphosphorane. The resulting condensation yields an unsaturated alkylene bridge, which is then hydrogenated to yield product 9 compounds above. The advantage of the Wittig approach is that it is preferable for synthesis of compounds of formula 9 wherein n is 2 or more, i.e. with longer alkylene bridges. It is useful when n=1. Also, the Wittig approach is preferable for compounds of Formula I having labile substituents on the heterocycle, particularly halogens.

A third, more lengthy method of making carba derivatives also exists. Briefly, nitrile (4) is hydrolyzed with acid to the corresponding carboxylic acid. Then a three step conversion to an alcohol is performed, followed by oxidation to aldehyde 7. Condensation of the aryl/heterocycle ring is then carried out with the resulting aldehyde as described above.

2-Thio derivatives (11) are typically prepared by thiolating product 5 above (or its OH-protected analog) with appropriate reagent such as t-butylmercaptan, to yield

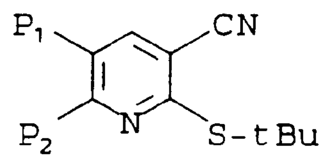

10

This compound now has a protecting group for the sulfur at the 2-position. The rest of the molecule can be constructed, and, as a final step, dealkylation in pyridine hydrochloride with heat results in the formation of:

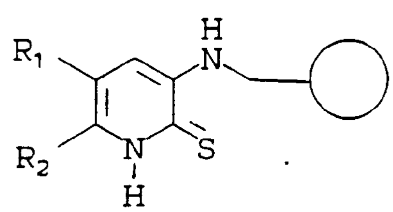

. 11

Direct thiolation with lawesson's Reagent is also useful for preparing 2-thio derivatives.

A procedure avails ether linkages at the 3-position of pyridone ring (12). Product 1 above is transformed in three steps into intermediate 12,

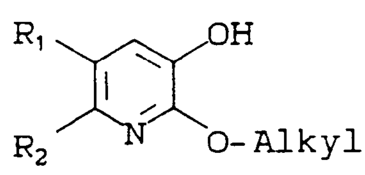

12

which is subseqently condensed with a halogenated aryl or halogenated heterocycle, in the presence of a base such as sodium hydride to yield

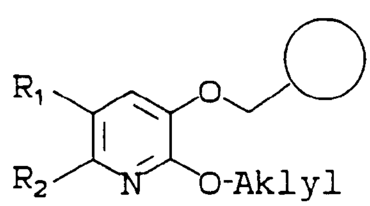

, 13

which is dealkylated with pyridine hydrochloride to yield

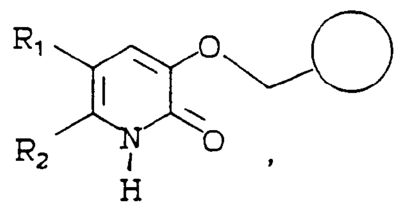

, 14

Modification by conventional methodology permits the preparation of the thio analog:

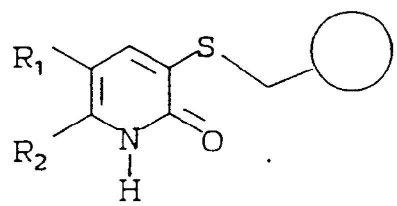

. 15

Dealkylation is conveniently achieved by heating with KI in glacial acetic acid.

## ATCC DEPOSIT

Before the U.S. filing date of the present application, a sample of the microorganism Actinoplanacete sp. (Merck Culture Collection MA 6559) was deposited at the American Type Culture Collection (ATCC), 12301 Parklawn Drive, Rockville MD 20852. The culture access designation is 53771. This deposit will be maintained in the ATCC for at least 30 years.

It should be understood that the availability of a deposit does not constitute a license to practice the subject invention in derogation of patent rights granted by government action.

## GENERAL CHARACTERISTICS OF ATCC 53771

The physical characteristics and taxonomy, including morphological, cultural, biological and physiological characteristics are briefly described hereinbelow.

On the basis of the taxonomic analysis performed thus far, the culture has tentatively been assigned to the order Actionmycetales and to the family Actinoplanacea.

This culture grows well on routine media including trypticase soy agar (28° and 37°C), yeast malt extract agar, glycerol asparagine agar, inorganic salt starch agar, oatmeal agar, Czapek Dox, Czapek solution agar and peptone agar, and Bennett's agar, all at 28°C.

Morpholgy - This culture grows as a branched filamentous mycelium with a diameter of 0.2 -0.4 microns. Colonies are opaque, raised, and erose. Colony texture is rubbery on yeast malt extract agar but tends to be butyrous on other media where significant fragmentation of the mycelium is observed. The colony surface tends to be powdery in appearance. No diffusable pigments were observed.

Sporangia - are predominantly spherical and range in size from 4 - 25 microns in diameter. Sporangia are generally visible by 21 days and tend to coalesce on glycerol asparagine agar. Spores are rod shaped with blunt ends (0.76 x 1.9 microns), non-motile and occur in long, unbranched chains of up to 150 microns in length.

## Cultural characteristics of ATCC 53771

### Yeast Extract-Malt Extract Agar (ISP Medium 2)

Vegetative mycelium is hyaline to yellow, aerial mycelium develops in 24 - 72 hours and is-buff to rose-pink and powdery in appearance. The reverse side is tan to reddish brown.

Oatmeal Agar (ISP Medium 3)

Vegetative mycelium is hyaline to yellow, the reverse side is hyaline to tan. Aerial growth is white to light rose-beige and powdery in appearance.

Inorganic Salts-Starch Agar (ISP Medium 4)

Light growth, scant aerial mycelium. Vegetative growth is hyaline and highly fragmented Clearing of starch occurs at periphery of colonies noted by 7 d.

Glycerol Asparagine Agar (ISP Medium 5)

Vegetative growth is hyaline to yellow, the reverse side is hyaline to cinnamon brown. Aerial mycelium is powdery and white to rose-pink.

Peptide-Iron-Yeast Extract Agar (ISP Medium 6)

Vegetative growth is tan. No aerial growth observed, no melanoid pigments produced .

Tyrosine Agar (ISP Medium 7)

Vegetative growth is tan becoming deep purple as culture ages. Aerial mycelium is velvety to grayed rose-beige.

Czapek-Dox Agar

Vegetative growth is tan with a pink tone as the culture ages. Aerial mycelia are short and matted with a moist appearance.

In general, the compounds L-697,913 and L-697,914 and others can be produced by culturing (fermenting) the ATCC 53771 strain with L-696,040 or other appropriate substrates in an aqueous nutrient medium containing sources of assimilable carbon and nitrogen, preferably under submerged aerobic conditions (e.g. shaking culture, submerged culture, etc.). The aqueous medium is preferably maintained at a pH of about 7 at the initiation and termination (harvest) of the fermentation process. A higher pH leads to substantial and/or total loss of product. The desired pH may be maintained by the use of a buffer such as morpholinoethanesulfonic acid (MES), morpholinopropanesulfonic acid (MOPS), and the like, or by choice of nutrient materials which inherently possess buffering properties, such as production media described hereinbelow.

The preferred sources of carbon in the nutrient medium are carbohydrates such as glucose, xylose, galactose, glycerin, starch, dextrin, and the like. Other sources which may be included are maltose, rhamnose, raffinose, arabinose, mannose, salicin, sodium succinate, and the like.

The preferred sources of nitrogen are yeast extract, meat extract, peptone, gluten meal, cottonseed meal, soybean meal and other vegetable meals (partially or totally defatted), casein hydrolysates, soybean hydrolysates, and yeast hydrolysates, corn steep liquor, dried yeast, wheat germ, feather meal, peanut powder, distiller's solubles, etc., as well as inorganic and organic nitrogen compounds such as ammonium salts (e.g. ammonium nitrate, ammonium sulfate, ammonium phosphate, etc.), urea, amino acids, and the like.

The carbon and nitrogen sources, though advantageously employed in combination, need not be used in their pure form, because less pure materials which contain traces of growth factors and considerable quantities of mineral nutrients, are also suitable for use. When desired, there may be added to the medium mineral salts such as sodium or calcium carbonate, sodium or potassium phosphate, sodium or potassium chloride, sodium or potassium iodide, magnesium salts, copper salts, cobalt salts, and the like. If necessary, especially when the culture medium foams seriously, a defoaming agent, such as liquid paraffin, fatty oil, plant oil, mineral oil or silicone may be added.

The L-696,040 can be obtained by synthetic organic procedures, as described elsewhere in this application.

As to the conditions for the production of L-697,913 or L-697,914 in massive amounts, submerged aerobic cultural conditions are preferred. For the production in small amounts, a shaking or surface culture in a flask or bottle is employed. Furthermore, when the growth is carried out in large tanks, it is preferable to use the vegetative form of the organism for inoculation in the production tanks in order to avoid growth lag in the process of production. Accordingly, it is desirable first to produce a vegetative inoculum of the organism by inoculating a relatively small quantity of culture medium with spores or mycelia of the organism produced in a "slant" and

culturing said inoculated medium, also called the "seed medium", and then to transfer the cultured vegetative inoculum aseptically to large tanks. The fermentation medium, in which the inoculum is produced, is generally autoclaved to sterilize the medium prior to inoculation. The pH of the medium is generally adjusted to about 7.0 prior to the autoclaving step by suitable addition of an acid or base, preferably in the form of a buffering solution.

Agitation and aeration of the culture mixture may be accomplished in a variety of ways. Agitation may be provided by a propeller or similar mechanical agitation equipment, by revolving or shaking the fermentor, by various pumping equipment or by the passage of sterile air through the medium. Aeration may be effected by passing sterile air through the fermentation mixture.

The fermentation is usually conducted at a temperature between about 20°C and 40°C, preferably 25-35°C, for a period of about 10 hours to 20 hours, which may be varied according to fermentation conditions and scales. Preferably, the production cultures are incubated for about 17 hours at 27°C on a rotary shaker operating at 220 rpm, wherein the pH of the fermentation medium is maintained at 7.0 to harvest.

Preferred culturing/production media for carrying out the fermentation include the following media:

| Seed Medium A | g/l |
|---|---|
| Dextrose | 1.0 |
| Dextrin | 10.0 |
| Beef Extract | 3.0 |
| Ardamine pH | 5.0 |
| NZ Amine Type E | 5.0 |
| $MgSO_4 \cdot 7H_2O$ | 0.05 |
| $K_2HPO_4$ | 0.37 |
| Adjusted pH to 7.1 | |
| Add $CaCO_3$ 0.5 g/l | |

| Transformation Medium B | g/l |
|---|---|
| Glucose | 10 |
| Hycase SF | 2 |
| Beef Extract | 1 |
| Corn Steep Liquor | 3 |
| Adjusted pH to 7.0 | |

| Transformation Medium C | g/l |
|---|---|
| Mannitol | 5 |
| Glycerol | 5 |
| Hycase SF | 2 |
| Beef extract | 1 |
| Corn Steep Liquor | 3 |
| Adjust pH to 7.0 | |

The produced L-697,913 or L-697,914 can be recovered from the culture medium by conventional means which are commonly used for the recovery of other known biologically active substances. The substances produced are found in the cultured mycelium and filtrate, and accordingly can be isolated and purified from the mycelium and the filtrate, which are obtained by filtering or centrifuging the cultured broth, by a conventional method such as concentration under reduced pressure, lyophilization, extraction with a conventional solvent, such as methylene chloride or methanol and the like, pH adjustment, treatment with a conventional resin (e.g. anion or cation exchange resin, non-ionic adsorption resin, etc.), treatment with a conventional adsorbent (e.g. activated charcoal, silicic acid, silica gel, cellulose, alumina, etc.), crystallization, recrystallization, and the like. A preferred method is solvent extraction, particularly using methylene chloride.

FORMULATIONS

The compounds of the present invention may be administered orally, parenterally (including subcutaneous injections, intravenous, intramuscular, intrasternal injection or infusion techniques), by inhalation spray, or rectally, in dosage unit formulations containing conventional non-toxic pharmaceutically-acceptable carriers, adjuvants and vehicles.

Thus, in accordance with the present invention there is further provided a method of treating and a pharmaceutical composition for treating HIV infection and AIDS. The treatment involves administering to a patient in need of such treatment a pharmaceutical composition comprising a pharmaceutical carrier and a therapeutically-effective amount of a compound of the present invention.

These pharmaceutical compositions may be in the form of orally-administrable suspensions or tablets; nasal sprays; sterile injectable preparations, for example, as sterile injectable aqueous or oleagenous suspensions or suppositories.

When administered orally as a suspension, these compositions are prepared according to techniques well-known in the art of pharmaceutical formulation and may contain microcrystalline cellulose for imparting bulk, alginic acid or sodium alginate as a suspending agent, methylcellulose as a viscosity enhancer, and sweetners/flavoring agents known in the art. As immediate release tablets, these compositions may contain microcrystalline cellulose, dicalcium phosphate, starch, magnesium stearate and lactose and/or other excipients, binders, extenders, disintegrants, diluents and lubricants known in the art.

When administered by nasal aerosol or inhalation, these compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art.

The injectable solutions or suspensions may be formulated according to known art, using suitable non-toxic, parenterally-acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono- or diglycerides, and fatty acids, including oleic acid.

When rectally administered in the form of suppositories, these compositions may be prepared by mixing the drug with a suitable non-irritating excipient, such as cocoa butter, synthetic glyceride esters or polyethylene glycols, which are solid at ordinary temperatures, but liquify and/or dissolve in the rectal cavity to release the drug.

The compounds of this invention can be administered orally to humans in a dosage range of 1 to 100 mg/kg body weight in divided doses. One preferred dosage range is 1 to 10 mg/kg body weight orally in divided doses. Another preferred dosage range is 1 to 20 mg/kg body weight orally in divided doses. It will be understood, however, that the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

The present invention is also directed to combinations of the HIV reverse transcriptase inhibitor compounds with one or more agents useful in the treatment of AIDS. For example, the compounds of this invention may be effectively administered, whether at periods of pre-exposure and/or post-exposure, in combination with effective amounts of the AIDS antivirals, immunomodulators, anti-infectives, such as those in the following Table.

## TABLE

### ANTIVIRALS

| Drug Name | Manufacturer | Indication |
|---|---|---|
| AL-721 | Ethigen (Los Angeles, CA) | ARC, PGL HIV positive, AIDS |
| Recombinant Human Interferon Beta | Triton Biosciences (Almeda, CA) | AIDS, Kaposi's sarcoma, ARC |
| Acemannan | Carrington Labs (Irving, TX) | ARC (See also immunomodulators) |
| Cytovene | Syntex | sight threatening CMV |
| Ganciclovir | (Palo Alto, CA) | peripheral CMV retinitis |

| Drug Name | Manufacturer | Indication |
|---|---|---|
| d4T Didehydrodeoxy-thymidine | Bristol-Myers (New York, NY) | AIDS, ARC |
| ddI Dideoxyinosine | Bristol-Myers (New York, NY) | AIDS, ARC |
| EL10 | Elan Corp, PLC (Gainesville, GA) | HIV infection (See also immunomodulators) |
| Trisodium Phosphonoformate | Astra Pharm. Products, Inc. (Westborough, MA) | CMV retinitis, HIV infection, other CMV infections |
| Dideoxycytidine; ddC | Hoffman-La Roche (Nutley, NJ) | AIDS, ARC |
| Novapren | Novaferon Labs, Inc. (Akron, OH) Diapren, Inc. (Roseville, MN, marketer) | HIV inhibitor |
| Peptide T Octapeptide Sequence | Peninsula Labs (Belmont, CA) | AIDS |
| Zidovudine; AZT | Burroughs Wellcome (Rsch. Triangle Park, NC) | AIDS, adv, ARC pediatric AIDS, Kaposi's sarcoma, asymptomatic HIV |

| Drug Name | Manufacturer | Indication |
|---|---|---|
| | | infection, less severe HIV disease, neurological involvement, in combination w/other therapies, post-exposure prophylaxis in health care workers |
| Ansamycin LM 427 | Adria Laboratories (Dublin, OH) Erbamont (Stamford, CT) | ARC |
| Dextran Sulfate | Ueno Fine Chem. Ind. Ltd. (Osaka, Japan) | AIDS, ARC, HIV positive asymptomatic |
| Virazole Ribavirin | Viratek/ICN (Costa Mesa, CA) | asymptomatic HIV positive, LAS, ARC |
| Alpha Interferon | Burroughs Wellcome (Rsch. Triangle Park, NC) | Kaposi's sarcoma, HIV in combination w/Retrovir |
| Acyclovir | Burroughs Wellcome | AIDS, ARC, asymptomatic HIV positive, in combination with AZT. |

IMMUNO-MODULATORS

| Drug Name | Manufacturer | Indication |
| --- | --- | --- |
| Antibody which neutralizes pH labile alpha aberrant Interferon in an immuno-adsorption column | Advanced Biotherapy Concepts (Rockville, MD) | AIDS, ARC |
| AS-101 | Wyeth-Ayerst Labs. (Philadelphia, PA) | AIDS |
| Bropirimine | Upjohn (Kalamazoo, MI) | advanced AIDS |
| Acemannan | Carrington Labs, Inc. (Irving, TX) | AIDS, ARC (See also antivirals) |
| CL246,738 | American Cyanamid (Pearl River, NY) Lederle Labs (Wayne, NJ) | AIDS, Kaposi's sarcoma |
| EL10 | Elan Corp, PLC (Gainesville, GA) | HIV infection (See also antivirals) |
| Gamma Interferon | Genentech (S. San Francisco, CA) | ARC, in combination w/TNF (tumor necrosis factor) |

| Drug Name | Manufacturer | Indication |
| --- | --- | --- |
| Granulocyte Macrophage Colony Stimulating Factor | Genetics Institute (Cambridge, MA) Sandoz (East Hanover, NJ) | AIDS |
| Granulocyte Macrophage Colony Stimulating Factor | Hoeschst-Roussel (Somerville, NJ) Immunex (Seattle, WA) | AIDS |
| Granulocyte Macrophage Colony Stimulating Factor | Schering-Plough (Madison, NJ) | AIDS<br>AIDS, in combination w/AZT |
| HIV Core Particle Immunostimulant | Rorer (Ft. Washington, PA) | seropositive HIV |
| IL-2 Interleukin-2 | Cetus (Emerycille, CA) | AIDS, in combination w/AZT |
| IL-2 Interleukin-2 | Hoffman-La Roche (Nutley, NJ) Immunex | AIDS, ARC, HIV, in combination w/AZT |
| Immune Globulin Intravenous (human) | Cutter Biological (Berkeley, CA) | pediatric AIDS, in combination w/AZT |

| Drug Name | Manufacturer | Indication |
|---|---|---|
| IMREG-1 | Imreg (New Orleans, LA) | AIDS, Kaposi's sarcoma, ARC, PGL |
| IMREG-2 | Imreg (New Orleans, LA) | AIDS, Kaposi's sarcoma, ARC, PGL |
| Imuthiol Diethyl Dithio Carbamate | Merieux Institute (Miami, FL) | AIDS, ARC |
| Alpha-2 Interferon | Schering Plough (Madison, NJ) | Kaposi's sarcoma w/AZT: AIDS |
| Methionine-Enkephalin | TNI Pharmaceutical (Chicago, IL) | AIDS, ARC |
| MTP-PE Muramyl-Tripeptide | Ciba-Geigy Corp. (Summit, NJ) | Kaposi's sarcoma |
| Granulocyte Colony Stimulating Factor | Amgen (Thousand Oaks, CA) | AIDS, in combination w/AZT |
| rCD4 Recombinant Soluble Human CD4 | Genentech (S. San Francisco, CA) | AIDS, ARC |
| rCD4-IgG hybrids | | AIDS, ARC |

| Drug Name | Manufacturer | Indication |
|---|---|---|
| Recombinant Soluble Human CD4 | Biogen (Cambridge, MA) | AIDS, ARC |
| Interferon Alfa 2a | Hoffman-La Roche (Nutley, NJ) | Kaposi's sarcoma AIDS, ARC, in combination w/AZT |
| SK&F106528 Soluble T4 | Smith, Kline & French Laboratories (Philadelphia, PA) | HIV infection |
| Thymopentin | Immunobiology Research Institute (Annandale, NJ) | HIV infection |
| Tumor Necrosis Factor; TNF | Genentech (S. San Francisco, CA) | ARC, in combination w/gamma Interferon |

ANTI-INFECTIVES

| Drug Name | Manufacturer | Indication |
|---|---|---|
| Clindamycin with Primaquine | Upjohn (Kalamazoo, MI) | PCP |
| Fluconazole | Pfizer (New York, NY) | cryptococcal meningitis, candidiasis |
| Pastille Nystatin Pastille | Squibb Corp. (Princeton, NJ) | prevention of oral candidiasis |

| Drug Name | Manufacturer | Indication |
|---|---|---|
| Ornidyl Eflornithine | Merrell Dow (Cincinnati, OH) | PCP |
| Pentamidine Isethionate (IM & IV) | LyphoMed (Rosemont, IL) | PCP treatment |
| Piritrexim | Burroughs Wellcome (Rsch. Triangle Park, NC) | PCP treatment |
| Pentamidine isethionate for inhalation | Fisons Corporation (Bedford, MA) | PCP prophylaxis |
| Spiramycin | Phone-Poulenc Pharmaceuticals (Princeton, NJ) | cryptosporidial diarrhea |
| Intraconazole-R51211 | Janssen Pharm. (Piscataway, NJ) | histoplasmosis; cryptococcal meningitis |
| Trimetrexate | Warner-Lambert | PCP |

OTHER

| Drug Name | Manufacturer | Indication |
|---|---|---|
| Recombinant Human Erythropoietin | Ortho Pharm. Corp. (Raritan, NJ) | severe anemia assoc. and AZT therapy |

| Drug Name | Manufacturer | Indication |
|---|---|---|
| Megestrol Acetate | Bristol-Myers (New York, NY) | treatment of anorexia assoc. w/AIDS |
| Total Enteral Nutrition | Norwich Eaton Pharmaceuticals (Norwich, NY) | diarrhea and malabsorption related |

It will be understood that the scope of combinations of the compounds of this invention with AIDS antivirals, immunomodulators, anti-infectives or vaccines is not limited to the list in the above Table, but includes in principle any combination with any pharmaceutical composition useful for the treatment of AIDS.

EXAMPLE 1

Preparation of Parent Compound, 3-[(2-Benzoxazolylmethyl)amino]-5-ethyl-6-methyl-2-(1H)-pyridinone, L-696,040

Step A) Preparation of 5-ethyl-6-methyl-3-nitro-2-(1H)-pyridinone

A mixture of 2-ethyl-3-oxobutanal, sodium salt (7.5 g, 55 mmol), nitroacetamide (6.6 g, 63 mmol), aqueous piperidinium acetate (4.4 mL) [prepared from glacial acetic acid (42 mL), water (100 mL) and piperidine (72 mL)] in water (45 mL) was stirred at room temperature for 22 hours. The yellow precipitate was collected by filtration and air dried to yield 8.0 g (80%) of 5-ethyl-6-methyl-3-nitro-2-(1H)-pyridinone.

Step B) Preparation of 3-amino-5-ethyl-6-methyl-2-(1H)-pyridinone

A yellow solution of the 5-ethyl-6-methyl-3-nitro-2-(1H)-pyidinone (10 g, 55 mmol) in a mixture of methanol and tetrahydrofuran (100 mL, 1:1 v/v) was reduced catalytically in the presence of 7% palladium on charcoal (0.7 g ) under an atmosphere of hydrogen (50 psi) at room temperature over a period of 3.5 hours. The resultant mixture was filtered through a small pad of Celite. The filtrate was concentrated under reduced pressure (15 torr) to provide 5.7 g (68%) of the corresponding aminopyridone.

Step C) Preparation of 3-[(2-Benzoxazolymethyl)amino]-5-ethyl-6-methyl-2-(1H)-pyridinone, L-696,040

A solution of 3-amino-5-ehtyl-6-methyl-2-(1H)-pyridinone (152 mg, 1.0 mmol), 2-chloromethyl-1,3-benzoxazole (1.07 mmol) and triethylamine (0.14 mL, 1.0 mmol) in acetonitrile (10 mmL) was stirred at reflux for 24 hours. After concentrating under reduced pressure, the residue was flash chromatographed over silica gel. Elution with 5% MeOH- 95% $CHCl_3$ gave 132 mg of product which was recrystallized from EtOH-water to give 95 mg of analytically pure product, mp 202-203°C, with initial melting at 179° followed by resolidification.
Anal. Calcd for $C_{16}H_{17}N_3O_2$:

C, 67.83, H, 6.05; N, 14.83

Found: C, 67.71; H, 6.03; N, 14.76.

EXAMPLE 2

Fermentation of L-696,040

A frozen vial (2.0 ml) of culture MA6559 (ATCC 53771) was used to inoculate a 250 ml baffled shake flask containing 50 ml of seed medium A. The seed flask was incubated on a rotary shaker (220 rpm) at 27°C for 24 hours. A 1 ml aliquot of the developed seed was used to inoculate a 50 ml non-baffled flask containing 10 ml of transformation medium B; L-696,040 in DMSO was added to the fermentation at 0 hour to achieve a final

concentration of 0.05 mg/ml. The shake flask contents were subsequently incubated at 27°C on rotary shaker for two days. The resultant whole both was extracted as described in the next example.

| Media | Seed Medium A | g/l |
|---|---|---|
| | Dextrose | 1.0 |
| | Dextrin | 10.0 |
| | Beef Extract | 3.0 |
| | Ardamine pH | 5.0 |
| | NZ Amine Type E | 5.0 |
| | $MgSO_4 \cdot 7H_2O$ | 0.05 |
| | $K_2HPO_4$ | 0.3 |
| | Adjust pH to 7.1 | |
| | Add to $CaCO_3$ 0.5 g/l | |

| Transformation Medium B | g/l |
|---|---|
| Glucose | 10 |
| Hycase SF | 2 |
| Beef Extract | 1 |
| Corn Steep Liquor | 3 |
| Adjust pH to 7.0 | |

EXAMPLE 3

Isolation and Purification of Biotransformed Products

The whole broth (50 ml) of the preceeding example was adjusted to pH 9 and extracted with methylene chloride (3x50 ml). Methylene chloride extracts were combined and concentrated under vacuum to an oily residue. The residue was dissolved in methanol and subjected to high performance liquid chromatography (HPLC). HPLC was carried out on Whatman Partisil 10 ODS-3, 9.4 mm x 25 cm at room temperature and monitored at 250 nm. The column was developed at 3 ml/min with a linear gradient from 0.1% aqueous TFA-$CH_3CN$, 75:25, to 0.1% aqueous TFA-$CH_3CN$, 20:80, in 30 minutes. The compounds were collected during repeated injections of the above described extract. The fractions at retention time, 10.5 and 13.5 minutes, were pooled, adjusted to pH 9, respectively, and evaporated to remove acetonitrile. The compounds were then extracted into the methylene chloride to yield 200 μg of L-697,913 and 450 μg of L-697,914, respectively.

EXAMPLE 4

Structure Determination

The structures of two biotransformation products of L-696,040 (compound 1) were determined by NMR. Metabolite 2 was shown to result from hydroxylation of the methyl group, while metabolite 3 from hydroxylation at $CH_2$ of the ethyl side chain.

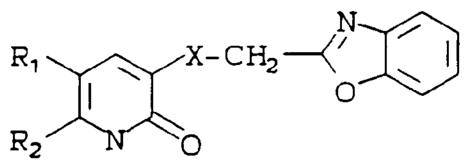

| Compd | X | $R_1$ | $R_2$ | Sample |
|---|---|---|---|---|
| 1. | NH | $CH_2CH_3$ | $CH_3$ | L-696,040 |
| 2. | NH | $CH_2CH_3$ | $CH_2OH$ | L-697,914 |
| 3. | NH | $CH(OH)CH_3$ | $CH_3$ | L-697,913 |

Hydroxylation of the methyl group in 2 was indicated by the disappearance of methyl singlet near 2.2 ppm of parent compound 1 and the appearance instead of a new singlet at ~4.45 ppm ascribable to the $CH_2OH$ protons.

Hydroxylation of C-2 of the ethyl side chain in 3 was evident from the appearance of the terminal methyl group as a doublet (rather than a triplet), and the appearance of a CHOH quartet near 4.8 ppm instead of the methylene quartet at ~2.25 ppm in the parent compound.

## EXAMPLE 5

### Organic Synthesis of L-697,913

Steps A-C of Example 1 are repeated, except that 2-ethyl-3-oxobutanal is substituted with 2-(1-benzyloxy)ethyl-3-oxobutanal. The benzyloxy protecting group is removed in a final step of hydrogenation over palladium on carbon, yielding L-697,913.

## EXAMPLE 6

### Organic Synthesis of L-697,914

Steps A-C of Example 1 are repeated, except that 2-ethyl-3-oxobutanal is substituted with 2-ethyl-3-oxo-4-benzyloxybutanal. The benzyloxy protecting group is removed in a final step of hydrogenation over palladium on carbon, yielding L-697,914.

## EXAMPLE 7

### 3-[2-(Benzoxazol-2-yl)ethyl]-5-ethyl-6-hydroxy-methylpyridin-2(1H)-one

Step 1: Prep of 4-Benzyloxy-3-oxo-2-ethylbutanal

A solution of 90% pure 1-(N-morpholino)-1-butene (9.3gm, 60mmol) and triethylamine (8.4mL, 60mmol) in tetrahydrofuran (85mL), under a nitrogen atmosphere, was warmed to 70°C and benzyloxyacetyl chloride (9.45mL, 60mmol) was added dropwise via a syringe. The cloudy yellow solution was warmed for 45 minutes and then cooled to room temperature. An aqueous solution of 10% HCl (75mL) was added and the two phase mixture was stirred for 1 hour. This mixture was partitioned into methylene chloride and the organic layer was separated, dried ($Na_2SO_4$) filtered and the solvent evaporated to give a yellow oil (14.7g) which contained 80% desired product. This oil was used as is.

Step 2: Prep. of 3-Cyano-5-ethyl-6-benzyloxymethylpyridin-2(1H)-one

To a solution of crude 4-benzyloxy-3-oxoethylbutanal (6.4gm, 24mmol) and malononitrile (2.22g, 33mmol) in ethanol (30mL) was added acetic acid (2.0mL, 35mmol), followed by dropwise addition of piperidine (2.37 mL, 24mmol) to give a dark reddish brown solution. After stirring for 14 hours a copious precipitate formed. This mixture was warmed at 70°C for 15 hours and then allowed to cool to room temperature. This blackish mixture was diluted with ethanol and the precipitated product was filtered, rinsed with ethanol and diethyl ether to give off-white product (1.46g), mp: 141-143°C. Additional material was obtained by evaporation of filtrate. Extraction of the residue into choroform was conducted, and after washing this extract with saturated aqueous $NaHCO_3$, filtration through a pad of charcoal was performed. This amber solution was evaporated and the residue triturated with diethyl ether to give additional product (.47g). The estimated yield of this reaction was 30%.

Step 3: Prep. of 2-Benzyloxy-3-cyano-5-ethyl-6-benzyloxymethylpyridine

To a partial suspension of 3-cyano-5-ethyl-6-benzyloxymethylpyridin-2(1H)-one (1.36g, 5.08mmol) in dry benzene (20mL) was added benzyl bromide (0.80mL, 6.7mmol) and then silver carbonate (1.43g, 5.2mmol). This mixture was covered with aluminum foil and allowed to stir at room temperature. After 24 hours, the reaction was estimated to be -80% complete. Additional silver carbonate (.40g, 1.45mmol) was added and the mixture was stirred for another 24 hours until complete. The silver salts were removed by filtration, rinsed with benzene and the combined benzene washings were evaporated to give pure product as an oil (2.09g, quantitative).

Step 4: Prep. of 2-benzyloxy-5-ethyl-6-benzyloxymethyl nictinaldehyde

To a solution of 2-benzyloxy-3-cyano-5-ethyl-6-benzyloxymethyl-pyridine (2.57g, 6.2mmol) in dry toluene (10mL), under a nitrogen atmosphere in an ice/acetone bath, was added dropwise a solution of 1.5M diisobutylaluminum hydride/toluene (4.6mL, 6.9mmol). After stirring at room temperature for 15 hours, the reaction was cautiously poured into 10% HCl (30mL), stirred for 0.5 hours and the product extracted into diethyl ether. The ethereal solution was dried, filtered thru a pad of charcoal and evaporated to give a pale yellow oil (1.71g). This oil was further purified by passing a benzene solution through a plug of silica gel to give pure product (1.44g, 55% yield) upon evaporation.

Step 5: Prep of 3-[2-(Benzoxazol-2-yl)ethenyl]-5-ethyl-6-benzyloxymethyl-2-benzyloxypyridine

To a suspension of [(benzoxazol-2-yl)methyl]triphenylphosphonium chloride (1.77g, 4.12mmol) in dry tetrahydrofuran (20mL), under a nitrogen atomosphere, was added 60% NaH/mineral oil (.42g, 10mmol). After 0.5 hour a solution of 2-benzyloxy-5-ethyl-6-benzyloxymethylnicotinaldehyde (1.44g, 4.0 mmol) in tetrahydrofuran (10mL) was added and the reaction mixture was refluxed for 23 hours. The reaction was cooled, neutralized with acetic acid and partitioned between water and chloroform. The chloroform layer was washed with $NaHCO_3$ solution, dried, filtered through a pad of charcoal and evaporated. The material was dissolved in chloroform and flash chromatographed through silica gel to give a pure product as a yellow oil (1.40g, 74% yield). The product was a mixture of cis and tran olefins (ratio ~0.37).

Step 6: Prep of 3-[2-(Benzoxazol-2-yl)ethyl]-5-ethyl-6-benzyloxymethylpyridine-2-(1H)-one

A solution of cis/trans 3-[2-(benzoxazol-2-yl)ethenyl]-5-ethyl-6-benzyloxymethyl-2-benzyloxypyridine (1.40g, 2.94mmol) in dry tetrahydrofuran (20mL) and methanol (75mL) containing 10%Pd on charcoal (215mg) as a catalyst was hydrogenated at atomosphere pressure for 20 hours. The catalyst was filtered off and the solution evaporated to give a white residue. This residue was triturated with diethyl ether to give pure product (.78gm, 69% yield), mp: 140-142°C.

Step 7: Prep of 3-[2-(Benzoxazol-2-yl)ethyl]-5-ethyl-6-hydroxymethylpyridine-2-(1H)-one

A solution of 3-[2-(benzoxazol-2-yl)ethyl]-5-ethyl-6-benzyloxymethylpyridin-2-(1H)-one (198mg, 0.51mmol) in dry methylene chloride (6mL) was cooled in an ice/acetone bath and 1M boron tribromide hexane (1.5mL, 1.5mmol) was added dropwise to give a white precipitate. The suspension was stirred for one hour and then the reaction was quenched by addition of saturated aqueous $NaHCO_3$ (10mL). After stirring for 0.5 hour, the product was extracted into $CH_2Cl_2$, dried, filtered and the solvent evaporated to give a solid. Trituration with diethyl ether gave product as a tan solid (138mg, 90% yield). Recrystallization from ethyl acetate afforded

a light yellow solid (100mg), mp: 155-156°C.

Anal. Calcd for $C_{17}H_{18}N_2O_3$: C, 68.44; H, 6.08; N, 9.39
Found: C, 68.15; H, 6.03; N, 9.07

EXAMPLE 8

(±)-3-[2-(Benzoxazol-2-yl)ethyl]-5-(1-hydroxyethyl)-6-methylpyridin-2(1H)-one

Step A: 2-Benzyloxy-5-acetyl-6-methylpyridin-3-carbonitrile

A mixture of 3-cyano-5-acetyl-6-methylpyridin-2(1H)-one (1.76g, 0.01 mol) (L. Mosti et al, J. Het. Chem. 22, 1503 (1985)), benzyl bromide (2.12g, 0.012 mol), silver carbonate (3.06g, 0.011 mol) in benzene was stirred at room temperature under a nitrogen atmosphere overnight, while protected from light. The following day the mixture was filtered, and the solids washed well with benzene. Evaporation of the filtrate gave the desired intermediate (2.47g, 92.8% yield), mp 94-99°C.

Anal.Calcd for$C_{16}H_{14}N_2O_2$ - MW 266.299
C, 72.17; H, 5.30; N 10.52;
Found: C, 71.97; H, 5.30; N, 10.19.

Step B: 2-Benzyloxy-5-(1-hydroxyethyl)-6-methylpyridin-3-carbonitrile

Sodium borohydride (0.35 g, 0.0092 mol) was added at room temperature to 2-benzyloxy-3-cyano-5-acetyl-6-methylpyridin-2(1H)-one (2.44g, 0.0092 mol) in ethanol (60mL). After 2 hours chloroform was added plus a few drops of acetic acid. After washing with water and brine, the chloroform solution was evaporated to give product (2.85g) without further purification.

Step C: 2-Benzyloxy-5-(1-hydroxyethyl)-6-methylpyridin-3-carboxaldehyde

To a mixture of 2-benzyloxy-5-(1-hydroxyethyl)-6-methylpyridin-3-carbonitrile (1.00g, 0.0037 mol) in toluene (25 mL), cooled in an ice-acetone bath, was added dropwise diisobutylaluminum hydride (1.5 M in toluene) (5.22 mL, 0.0078 mol). After stirring for one hour under nitrogen the mixture was worked up by quenching into a mixture of excess 1N hydrochloric acid and ice. Extraction with chloroform followed by washing with water, saturated sodium bicarbonate solution, and then brine, and evaporation, gave the desired product (0.091g, 90% yield).

Step D: (±)-3-[2-(Benzoxazol-2-yl)ethyl]-5-(1-hydroxyethyl)-6-methyl-2(1H)-pyridinone

The product of step C is then condensed with [(benzoxazol-2-yl)methyl]triphenylphosphonium chloride via the Wittig reaction, then treated with base, in accordance with the procedure and protocols of Example 7, Steps 5-7, yielding the title compounds, mp 185-187°C.
$^1$H-NMR (DMSO-$d_6$) 1.08 (d, 3H, J=6.6 Hz), 2.13 (s, 3H), 2.90 (m, 2H), 3.17 (t, 2H, J=6.9 Hz), 4.61 (m, 1H), 4.85 (d, 1H, J=3.9 Hz), 7.4 (m, 2H), 7.5 (m, 2H).

Anal. Calcd. for $C_{17}H_{18}N_2O_3$ - MW 298.34
C, 68.44; H, 6.08; N, 9.39;
Found: C, 68.16; H, 5.85; N, 9.43.

EXAMPLE 9

3-[2-(4,7-Dichlorobenzoxazol-2-yl)ethyl]-5-ethyl-6-hydroxymethylpyridin-2(1H)-one

Step A: Preparation of 2-benzyloxy-3[2-(4,7-dichlorobenzoxazol-2-yl)ethenyl]-5-ethyl-6-benzyloxymethylpyridine

Sodium hydride/mineral oil (60%, 50 mg, 1.2 mmol) was added to a suspension of (4,7-dichlorobenzoxazol-2-yl)methyltriphenylphosphonium chloride (515 mg, 1.03 mmol) in dry tetrahydrofuran (9 mL) under a nitrogen atmosphere. After .75 hours, 2-benzyloxy-5-ethyl-6-benzyloxymethylnicotinaldehyde (375 mg, 1.03 mmol) was added to the yellow suspension and the reaction mixture was refluxed for 1.5 hours. The reaction was cooled, diluted with chloroform, neutralized with a few drops of acetic acid, and washed with water, $NaHCO_3$ solution, dried ($Na_2SO_4$) and evaporated. This residue (1.05 g) was dissolved in benzene and flash chromatographed through a plug of silica gel. Appropriate fractions were combined and evaporated to give light oily product (430 mg, 76% yield). NMR indicated that this oil was a 2.3:1 mixture of trans/cis isomers.

Step B: Preparation of 3-[2-(4,7-Dichlorobenzoxazol-2-yl)ethyl]-5-ethyl-6-hydroxymethylpyridin-2(1H)-one

A trans/cis mixture of 2-benzyloxy-3-[2-(4,7-dichlorobenzoxazol-2-yl)ethenyl]-5-ethyl-6-benzyloxymethylpyridine (430 mg, 0.78 mmol) was dissolved in methanol (18 mL) and tetrahydrofuran (18 mL) containing 5% palladium/charcoal (154 mg) and hydrogenated overnight at atmospheric pressure. The catalyst was filtered and the solvent evaporated to give 285 mg of a mixture of products. This material was chromatographed on silica gel eluting with a 0.5-5% methanol/chloroform gradient. Appropriate fractions were combined and the solvent evaporated. This residue was triturated with diethyl ether to give 37 mg (13% yield) of product. Recrystallization from ethyl acetate gave 24 mg of analytically pure product, mp 151-152°C;
$^1H$ NMR (300 MHz, $CDCl_3$) $\delta$ 7.33 (1H, s), 7.24 (2H, ABq., J=6.3 Hz), 4.67 (2H, s), 3.35 (2H, t, J=7.3 Hz), 3.14 (2H, t, J=7.3 Hz), 2.36 (2H, ABq., J=7.5 Hz), 1.06 (3H, t, J=7.5 Hz).

Anal. calcd. for $C_{17}H_{16}Cl_2N_2O_3$:
C, 55.60; H, 4.39; N, 7.63.
Found: C, 55.99; H, 4.40; N, 7.67.

EXAMPLE 10

3-[2-(4,7-Dichlorobenzoxazol-2-yl)methylamino]-5-ethyl-6-hydroxymethylpyridin-2(1H)-one

Step A: Preparation of 3-nitro-5-ethyl-6-benzyloxymethylpyridin-2(1H)-one

Benzyloxy acetyl chloride (14.2mL, 0.09mol) in dry tetrahydrofuran (10mL) was added dropwise to a solution of 1-(N-morpholino)-1-butene (12.8g, 0.09mol) and triethylamine (12.6mL, 0.09mol) in dry tetrahydrofuran (120mL) warmed at 70°C under a nitrogen atmosphere. After 1.25 hours, the reaction was cooled to room temperature and nitro acetamide ammonium salt (12.0g, 0.099mol) was added, followed by the dropwise addition of acetic acid (11.4mL, 0.20mol). After stirring for 20-24 hours, the reaction was diluted with chloroform (150mL) and the solution washed with water, 10% HCl, dried ($Na_2SO_4$) and filtered through a pad of charcoal. The solvent was removed and the residue triturated with cold methanol. The crystalline yellow product was filtered, rinsed with methanol and diethyl ether to give 8.01g (31% yield), mp 157-158°C.

Anal. calcd. for $C_{15}H_{16}N_2O_4$:
C, 62.49; H, 5.59; N, 9.72.
Found: C, 62.11; H, 5.26; N, 9.68.

Step B: Preparation of 3-amino-5-ethyl-6-hydroxymethylpyridin-2(1H)-one

A solution of 3-nitro-5-ethyl-6-benzyloxymethylpyridin-2(1H)-one (576mg, 2.0mmol) in tetrahydrofuran

(15mL) and methanol (15mL) containing 10% palladium/charcoal (130mg) was hydrogenated at an atmospheric pressure of hydrogen, monitoring the progress by tlc. Additional catalyst was added in 100mg portions after day 2 and day 3. After 3-4 days, the catalyst was filtered and the solvents evaporated. The catalyst was vigorously washed with methanol/chloroform and combined solvents evaporated. The residue was triturated with methylene chloride and product collected by filtration to give 136 mg of 90% pure product. This material was used as is.

Step C: Preparation of 3-[2-(4,7-dichlorobenzoxazol-2-yl)methylamino]-5-ethyl-6-hydroxymethylpyridin-2(1H)-one

A suspension of crude 3-amino-5-ethyl-6-hydroxymethylpyridin-2(1H)-one (134mg, 0.80mmol) and 2-iodomethyl-4,7-dichlorobenzoxazole (275mg, 0.83mmol) in acetonnitrile (7mL) containing diisopropylethylamine (0.96mmol, 125mg) was warmed at 50°C for 4-5 hours. The precipitated product (165 mg) was removed by filtration and chromatographed on silica gel eluting with a 0-3.5% methanol/chloroform gradient. The appropriate fractions were combined, the solvents removed and the residue triturated with diethyl ether to give 95 mg (32% yield) of title compound, mp 193-195°C; $H^1$ NMR ($CDCl_3$,300MHz) δ 7.38 (2H,s), 6.50 (1H,s), 4.70 (2H,s), 4.49 (2H,s), 2.36 (2H, ABq, J = 7.6Hz) 1.10 (3H, t, J = 7.6 Hz).

Anal. calcd. for $C_{16}H_{15}Cl_2N_3O_3$:
C, 52.19; H, 4.11; N, 11.41.
Found: C, 52.11; H, 4.08; N, 11.03.

EXAMPLE 11

3-{N-[(2-Methoxy-5-ethyl-6-methylpyridin-3-yl)methyl]amino}-5-ethyl-6-hydroxymethylpyridin-2(1H)-one

A mixture of crude 3-amino-5-ethyl-6-hydroxymethylpyridin-2(1H)-one (100mg, 0.6mmol) and 2-methoxy-5-ethyl-6-methylnicotinaldehyde (110mg, 0.6mmol) in methanol (3mL) containing 1 drop of acetic acid was stirred at room temperature for 20 hours to give a copious yellow precipitate. Sodium cyanoborohydride (42mg, 0.65mmol) was added to this suspension. The precipitate dissolved and the product slowly precipitated over 3 hours. The precipitate was filtrated, rinsed with ethanol and diethyl ether and chromatographed on silica gel eluting with 0-3.5% methanol/chloroform gradient. The appropriate fractions were combined, the solvent evaporated and the residue triturated with diethyl ether to give 65mg of title compound, mp 204-205°C; $^1$H NMR ($CDCl_3$, 300MHz) δ 7.26 (1H,s), 6.15 (1H,s), 4.52 (2H,s), 4.23 (2H,s), 3.97 (3H,s), 2.51 (2H, ABq, J = 7.6Hz), 2.42 (3H,s), 2.33 (2H, ABq, J = 7.6Hz), 1.11 (3H, t, J = 7.6Hz), 1.06 (3H, t, J = 7.6Hz)

Anal. calcd. for $C_{18}H_{25}N_3O_3 \bullet 0.2H_2O$:
C, 64.53;H, 7.64; N, 12.54.
Found: C, 64.48; H, 7.47; N, 12.37.

EXAMPLE 12

3-[2-(2-methoxy-5-ethyl-6-methylpyridin-3-yl)ethyl]-5-ethyl-6-hydroxymethylpyridin-2(1H)-one

Step A: Preparation of 2-benzyloxy-3-hydroxymethyl-5-ethyl-6-benzyloxymethylpyridin

To a solution of 2-benzyloxy-5-ethyl-6-benzyloxymethylnicotinaldehyde (1.13 g, 3.13 mmol) in dry tetrahydrofuran (15 mL), under a nitrogen atmosphere was added lithium aluminum hydride (191 mg, 5.0 mmol). The reaction was stirred at room temperature for 2 hours and then warmed to 60°C for one hour. Saturated aqueous sodium sulfate was carefully added to the cooled reaction mixture. Powdered sodium sulfate was then added and the solution was filtered, the salts rinsed with chloroform and the combined solvents evaporated. The residue was triturated with cold hexane to give crystalline product (893 mg, 78% yield), mp 83.5-84.5°C.

Step B: Preparation of 3-[2-(2-methoxy-5-ethyl-6-methylpyridin-3-yl)ethenyl]-5-ethyl-6-benzyloxymethylpyridin-2(1H)-one

Thionyl chloride (0.37 ml, 5.1 mmol) was added to a solution of 2-benzyloxy-3-hydroxymethyl-5-ethyl-6-benzyloxymethylpyridine (844 mg, 2.32 mmol) in dry benzene (10 mL) and the reaction was warmed at 80°C for 2 hours. The solvent and excess thionyl chloride were removed under vacuum and the residue was triturated with diethyl ether to give 341 mg (50% yield) of 3-chloromethyl-5-ethyl-6-benzyloxymethylpyridin-2(1H)-one.

This material was dissolved in tetrahydrofuran (10 mL) and triphenyl phosphine hydrobromide (412 mg, 1.17 mmol) was added. This mixture was refluxed for 3 hours. The copious precipitate was collected by filtration, rinsed with diethyl ether to give 457 mg of a 2:1 mixture of triphenyl[5-ethyl-6-benzyloxymethylpyridin-2(1H)-on-3-yl]phosphonium bromide and 3-chloromethyl-5-ethyl-6-benzyloxymethylpyridin-2(1H)-one as indicated by nmr.

This mixture was suspended in dry tetrahydrofuran (10 mL), under a nitrogen atmosphere, and 80% sodium hydride/mineral oil (89 mg, 3.0 mmol) was added. After stirring at room temperature for 0.5 hour, 2-methoxy-5-ethyl-6-methylnicotinaldehyde (235 mg, 1.3 mmol) was added and the reaction mixture refluxed for 12-20 hours. The cooled reaction was diluted with chloroform (20 mL), acidified with acetic acid and then the separated organic layer was washed with water and saturated aqueous $NaHCO_3$. The organic layer was dried ($Na_2SO_4$), filtered through a charcoal plug and evaporated. This residue was flash chromatographed on silica gel eluting with a 0-1.5% methanol/chloroform gradient to give 638 mg of a 3:2 mixture of cis/trans 3-[2-(2-methoxy-5-ethyl-6-methylpyridin-3-yl)ethenyl]-5-ethyl-6-benzyloxymethylpyridin-2(1H)-one and triphenylphosphine oxide. This mixture was used as is.

Step C: Preparation of 3-[2-(2-methoxy-5-ethyl-6-methylpyridin-3-yl)ethyl]-5-ethyl-6-hydroxymethylpyridin-2(1H)-one

The mixture from Step B containing about 0.75 mmol of cis/trans 3-[2-(2-methoxy-5-ethyl-6-methylpyridin-3-yl)ethenyl]-5-ethyl-6-benzyloxymethylpyridin-2(1H)-one was dissolved in tetrahydrofuran (15 mL) and methanol (15 mL) containing 5% palladium/ charcoal (104 mg) and hydrogenated at atmospheric pressure for 55 hours. The catalyst was filtered and rinsed with chloroform/methanol and the combined organic solutions were evaporated. The residue was triturated with diethyl ether and crystalline by-product triphenyl phosphine oxide was removed by filtration. The resulting filtrate was evaporated to 435 mg of residue which was chromatographed on silica gel eluting with 0-3% methanol/chloroform gradient. The appropriate fractions were combined, the solvent evaporated and the residue triturated with diethyl ether to give 101 mg (41% yield) of title compound, mp 147-149°C; $^1$H-NMR ($CDCl_3$, 300 MHz) δ 7.06 (1H, s), 7.04 (1H, s), 4.66 (2H, s), 3.91 (3H, s), 2.78 (4H, br s), 2.48 (2H, ABq, J=7.6Hz), 2.37 (3H, s), 2.34 (2H, ABq, J=7.6Hz), 1.11 (3H, t, J=7.6Hz), 1.05 (3H, t, J=7.6Hz). Anal. Calc'd for $C_{19}H_{26}N_2O_3 \cdot 0.35H_2O$:

| | |
|---|---|
| | C, 67.77; H, 7.89; N, 8.32 |
| Found: | C, 67.71; H, 7.92; N, 8.16 |

EXAMPLE 13

Preparation of 3-[2-(4,7-Difluorobenzoxazol-2-yl)ethyl]-5-ethyl-6-hydroxymethylpyridin-2(1H)-one

Step A: Preparation of 2-Benzyloxy-6-benzyloxymethyl-3-[2-(4,7-difluorobenzoxazol-2-yl)ethenyl]-5-ethylpyridine

Sodium hydride (60% in mineral oil, 47 mg, 1.15 mmol) was added to a suspension of [(4,7-difluorobenzoxazol-2-yl)methyl]triphenylphosphonium chloride (512 mg, 1.1 mmol) (prepared by heating 2-chloromethyl-4,7-difluorobenzoxazole with an equimolar amount of triphenylphosphine in refluxing toluene for 15-25 hours) in dry THF (10 mL) under Ar at room temperature. After 15 minutes, 2-benzyloxy-6-benzyloxymethyl-5-ethylnicotinaldehyde (398 mg, 1.1 mmol) was added and the mixture heated at reflux for 15-25 hours. Upon cooling, $CHCl_3$ was added and after washing with brine the $CHCl_3$ solution was dried ($Na_2SO_4$), filtered and concentrated. The residue was flash chromatographed over silica gel. Pure olefin product was eluted with $CHCl_3$.

Step B: Preparation of 3-[2-(4,7-Difluorobenzoxazol-2-yl)ethyl]-5-ethyl-6-hydroxymethylpyridin-2(1H)-one

A solution of 2-benzyloxy-6-benzyloxymethyl-3-[2-(4,7-difluorobenzoxazol-2-yl)ethenyl]-5-ethylpyridine (410 mg, 0.82 mmol) in THF (20 mL) MeOH (30 mL) was hydrogenated over a 5% Pd/C catalyst at room temperature and atmospheric pressure for 8 hours until all of the starting material had been consumed. After filtering through diatomaceous earth and concentrating, the residue was flash chromatographed over silica gel. Elution with 3.5% MeOH-96.5% $CHCl_3$ gave product which was recrystallized from MeOH to 70 mg of analytically pure product, mp=182.5-84°.

Anal Calcd for $C_{17}H_{16}F_2N_2O_3$:

| | |
|---|---|
| | C, 61.08; H, 4.82; N, 8.38. |
| Found: | C, 60.88; H, 4.61; N, 8.21. |

EXAMPLE 14

Preparation of 5-Cyclpropyl-3-[((4,7-dichlorobenzoxazol-2-yl)methyl)amino]-6-hydroxymethylpyridin-2(1H)-one

Step A: Preparation of 6-Benzyloxymethyl-5-cyclopropyl-3-nitropyridin-2(1H)-one

2-Cyclopropylacetaldehyde (42 g, 0.50 mol) is added over 2 hours to a stirred mixture of morpholine (87 g, 1.0 mol) and $K_2CO_3$ (69 g, 0.50 mol) cooled to -5° to -10° under Ar. After addition is complete, the cooling bath is removed and the reaction stirred at room temperature overnight. Insoluble salts are removed by filtration and the filtrate distilled to give the enamine.

A solution of this enamine (15.3 g, 100 mmol) and triethylamine (12.6 mL, 90 mmol) in THF (125 mL) is heated to 70° under Ar and treated dropwise over 20 minutes with 2-benzyloxyacetyl chloride (14.2 mL, 90 mmol). After stirring at 70° for 1 hour, the mixture is cooled to room temperature and ammonium 2-nitroacetamide (12.1 g, 100 mmol) is added in one portion followed immediately by the dropwise addition of HOAc (11.4 mL) over 10 minutes. The heterogenous mixture is stirred at room temperature overnight. The reaction is diluted with $CHCl_3$ and washed with water and 1N HCl. After drying ($Na_2SO_4$), filtering and concentrating, the residue is triturated with MeOH and the insoluble solid washed with water and dried to give 6-benzyloxymethyl-5-cyclopropyl-3-nitropyridin-2(1H)-one.

Step B: Preparation of 3-Amino-5-cyclopropyl-6-hydroxymethylpyridin-2(1H)-one

A solution of 6-benzyloxymethyl-5-cyclopropyl-3-nitropyridin-2(1H)-one (1.42 g, 50 mmol) in EtOH (50 mL) and THF (50 mL) is hydrogenated over a 5% Pd/C catalyst at room temperature and 32 psi for 3 days. After filtering, solvents are removed under reduced pressure and the residue triturated first with $CHCl_3$ and then with $Et_2O$ to give 3-amino-5-cyclopropyl-6-hydroxymethylpyridin-2(1H)-one.

Step C: Preparation of 5-Cyclopropyl-3-[((4,7-dichlorobenzoxazol-2-yl)methyl)amino]-6-hydroxymethylpyridin-2(1H)-one

A mixture of 3-amino-5-cyclopropyl-6-hydroxymethylpyridin-2(1H)-one (1.1 g, 6.1 mmol), 2-chloromethyl-4,7-dichlorobenzoxazole (1.45 g , 6.1 mmol) and diisopropylethylamine (1.06 mL, 6.1 mmol) in acetonitrile (30 mL) is heated at reflux under Ar for 20 hours. After cooling to room temperature, solvents are removed under reduced pressure and the residue flash chromatographed over silica gel. Elution with 5% MeOH-95% $CHCl_3$ gives 5-cyclopropyl-3-[((4,7-dichlorobenzoxazol-2-yl)methyl)amino]-6-hydroxymethylpyridin-2(1H)-one.

EXAMPLE 15

Preparation of 3-[N-(5-Ethyl-2-methoxybenzyl)amino]-5-ethyl-6-hydroxymethylpyridin-2(1H)-one

Two drops of HOAc are added to a solution of 3-amino-5-ethyl-6-hydroxymethylpyridin-2(1H)-one (168 mg, 1.0 mmol) and 5-ethyl-2-methoxybenzaldehyde (162 mg, 1.0 mmol) in MeOH (5 mL) and the solution is stirred at room temperature for 2 hours. Sodium borohydride (378 mg, 10 mmol) is added portionwise to the suspension and the reaction stirred at room temperature for 30 minutes. After diluting with water and extracting with EtOAc,

the organic extract is washed with brine, dried ($Na_2SO_4$), filtered and concentrated. The residue is flash chromatographed over silica gel to give 3-[N-(5-ethyl-2-methoxybenzyl)amino]-5-ethyl-6-hydroxymethylpyridin-2(1H)-one.

EXAMPLE 16

Preparation of 3-[N-(4,5-Dimethyl-2-methoxybenzyl)amino]-5-ethyl-6-hydroxymethylpyridin-2(1H)-one

Two drops of HOAc are added to a solution of 3-amino-5-ethyl-6-hydroxymethylpyridin-2(1H)-one (168 mg, 1.0 mmol) and 4,5-dimethyl-2-methoxybenzaldehyde (162 mg, 1.0 mmol) in MeOH (5 mL) and the solution stirred at room temperature for 2 hours. Sodium borohydride (378 mg, 10 mmol) is added portionwise to the suspension and the reaction stirred at room temperature for 30 minutes. After diluting with water and extracting with EtOAc, the organic extract is washed with brine, dried ($Na_2SO_4$), filtered and concentrated. The residue is flash chromatographed over silica gel to give 3-[N-(4,5-dimethyl-2-methoxybenzyl)amino]-5-ethyl-6-hydroxymethylpyridin-2(1H)-one.

EXAMPLE 17

Preparation of 3-[2-(4,7-Dichlorobenzoxazol-2-yl)ethyl]-5-ethyl-6-hydroxymethylpyridin-2(1H)-thione

Step A: Preparation of 3-[2-(4,7-dichlorobenzoxazol-2-yl)-ethenyl]-5-ethyl-6-benzyloxymethylpyridin-2(1H)-one

A suspension of 2-benzyloxy-3-[2-(4,7-dichlorobenzoxazol-2-yl)ethynyl]-5-ethyl-6-benzyloxymethylpyridine (662 mg, 1.21 mmol) [see Example 9, Step A] in glacial acetic acid (25 mL) containing potassium iodide (802 mg, 4.83 mmol) was warmed at 60°C for 2-4 hours. The initial suspension dissolved and the yellow product precipitated. The cooled suspension was filtered to collect the product which was rinsed with acetic acid, water, methanol and diethyl ether to give 366 mg, mp 233-234°C. Re-filtration of the mother liquors yielded 167 mg of additional product (combined yield 96%). This material was used as is.

Step B: Preparation of 3-[2-(4,7-dichlorobenzoxazol-2-yl)ethyl]-5-ethyl-6-benzyloxymethylpyridin-2(1H)-one

A solution of 3-[2-(4,7-dichlorobenzoxazol-2-yl)ethenyl]-5-ethyl-6-benzyloxymetnylpyridin-2(1H)-one (250 mg, 0.55 mmol) in tetrahydrofuran (50 mL) containing 10% palladium/carbon (95 mg) was hydrogenated at atmospheric pressure for 15-24 hours. The catalyst was filtered off and the solvent evaporated. The residue was triturated with methanol to give 217 mg (86% yield) of product.

Step C: Preparation of 3-[2-(4,7-dichlorobenzoxazol-2-yl)ethyl]-5-ethyl-6-benzyloxymethylpyridin-2(1H)-thione

A suspension of 3-[2-(4,7-dichlorobenzoxazol-2-yl)ethyl]-5-ethyl-6-benzyloxymethylpyridin-2(1H)-one (214 mg, 0.468 mmol) in dry toluene (5 mL) containing 2,4-bis(4-methoxyphenyl)-1,3-dithia-2,4-diphosphetane-2,4-disulfide (Lawesson's Reagent) (231 m, 0.57 mmol) was warmed at 80°C for four hours to give a clear yellow solution. Upon cooling, methanol (5 mL) was added and the solvents evaporated. The residue was chromatographed on silica gel using a 0-0.5% $CH_3CH/CHCl_3$ gradient to give 190 mg (85% yield) of product, mp 187-190°C.

Step D: Preparation of 3-[2-(4,7-dichlorobenzoxazol-2-yl)ethyl]-5-ethyl-6-hydroxymethylpyridin-2(1H)-thione

A solution of 3-[2-(4,7-dichlorobenzoxazol-2-yl)ethyl]-5-ethyl-6-benzyloxymethylpyridin-2(1H)-thione (134 mg, 0.28 mmol) in methylene chloride (8 mL) was cooled to -10°C, under an inert atmosphere of nitrogen, and 1M boron trichloride/methylene chloride (0.75 mL, 0.75 mmol) was added dropwise. After 1-2 hours, aqueous $NaHCO_3$ (5 mL) was added slowly to the cloudy solution. The crude product was extracted into methylene chloride/methanol, dried ($Na_2SO_4$) and the solvents evaporated. The residue was chromatographed on silica gel and eluted with a 0-1.5% $CH_3OH/CHCl_3$ gradient. Appropriate fractions were combined, the solvent removed and the residue triturated with $CH_2Cl_2/Et_2O$ to give 34 mg of pure product (31% yield), mp 201-203°C. $^1H$ NMR ($CDCl_3$, 300 MHz) δ 7.59 (1H,s), 7.26 (2H, ABq, J=6 Hz), 4.87 (2H, s), 3.45 (4H, m), 2.46 (2H, ABq, J=7 Hz), 1.14 (3H, t, J=7 Hz).

Anal. calc'd for $C_{17}H_{16}Cl_2N_2O_2S \bullet 0.55\ H_2O$:
C, 51.92; H, 4.38; N, 7.12.
Found: C, 51.92; H, 4.12; N, 7.01.

EXAMPLE 18

Preparation of 3-[(4,7-Dichlorobenzoxazol-2-yl)methyl]amino-5-[1(R,S)-hydroxyethyl]-6-methylpyridin-2(1H)-one

Step A: 5-Acetyl-6-methyl-3-nitropyridin-2(1H)-one

A mixture of 3-dimethylaminomethylene-2,4-pentanedione (6.20 g, 0.04 mol) and 2-nitroacetamide (2.08 g, 0.04 mol) in tetrahydrofuran (80 ml) was heated at a bath temperature of 55-60°C undernitrogen for 48 hours. After evaporation to dryness, the residue was extracted with chloroform. Evaporation of the chloroform, followed by digesting the residue with 2-propanol, gave on filtration the title compound (1.83 g, 20.2% yield), mp 202-207°C. An analytical sample had mp 207-210°C;
$^1H$ NMR ($CDCl_3$) δ: 2.5 (s, 3H), 2.87 (s, 3H); 8.95 (s, 1H).

Anal. Calcd. for $C_8H_8N_2O_2$:
C, 48.98; H, 4.11; N, 14.28.
Found: C,49.19; H, 4.00; N, 14.19.

Step B: 3-Amino-5-(1-hydroxymethyl)-6-methylpyridin-2(1H)-one

A combination of 5-acetyl-6-methyl-3-nitropyridin-2(1H)-one (0.80 g, 0.0048 mol), 10% palladium on carbon (0.21 g) in ethanol (200 mL) was stirred under a balloon of hydrogen gas for 2 hours. Following filtration and evaporation, the residue was dissolved in ethanol (200 mL). A total of 0.55 g of sodium borohydride was added in portions and the reaction mixture stirred for 24 hours. After acidification with acetic acid and evaporation, the crude product was flushed twice with methanol. Chromatography was carried out on a silica gel column employing successively 5%-, 10%-, and 15%-methanol-methylene chloride mixtures for development. There was obtained 0.44 g (53% yield) of the title compound, mp >290°C.
$^1H$ NMR (DMSO-$d_6$) δ: 1.18 (d, J=6.3Hz, 3H); 2.05 (s, 3H); 4.63 (m, 1H); 4.72 (s, 2H); 4.78 (d, J=3.3Hz, 1H); 6.61 (s, 1H); 11.07 (s, 1H).

Anal. Calcd. for $C_8H_{12}N_2O_2 . 0.25\ H_2O$:
C, 55.64; H, 7.30; N, 16.22.
Found: C, 55.61; H, 7.30; N, 16.18.

Step 3: 3-[(4,7-Dichlorobenzoxazol-2-yl)methyl]amino-5-[1(R,S)-hydroxyethyl]-6-methylpyridin-2(1H)-one

A mixture of 3-amino-5-(1-hydroxyethyl)-6-methylpyridin-2(1H)-one (0.034 g, 0.0002 mol), 4,7-dichloro-2-iodomethylbenzoxazole (0.066 g,00.0002 mol), diisopropylethylamine (0.028 g, 0.00022 mol) in acetonitrile (5 mL) was heated under nitrogen in a bath maintained at 80°C for 16 hours. The product present after cooling was removed by filtration (0.040 g). Recrystallization was effected by dissolving in 50-50 methanol-methylene chloride (12 mL) with heating. After removing the methylene chloride under vacuum, the desired product crystallized (0.02 g, 27.2% yield), mp 236-238°C (dec).
$^1H$ NMR (DMSO-$d_6$) δ: 1.14 (d, J=6.3Hz, 3H); 2.06 (s, 3H); 4.61 (m, 1H); 4.67 (m, 2H); 4.76 (d, J=3.6Hz, 1H); 5.94 (t, J=6.3Hz, 1H); 6.53 (s, 1H); 7.50 (d, J=8.7Hz, 1H); 7.54 (d, J=8.7 Hz, 1H); 11.24 (s, 1H).

Anal. Calcd. for $C_{16}H_{15}Cl_2N_3O_3$:
C,52.19; H,4.11; N, 11.41.
Found: C, 51.74; H, 4.07; N,11.10.

EXAMPLE 19

Preparation of 3-[(4,7-Dichlorobenzoxazol-2-yl)methyl]amino-5-acetyl-6-methylpyridin-2(1H)-one

A mixture of 3-amino-5-acetyl-6-methylpyridin-2(1H)-one (0.033 g, 0.0002 mol), 4,7-dichloro-2-iodomethylbenzoxazole (0.066 g, 0.0002 mol), and diisopropylethylamine (0.028 g, 0.00022 mol) in acetonitrile (3 mL) was heated at reflux for 24 hours. After cooling, the solids obtained on filtration (0.014 g) had mp 300-304°C (dec). A second crop (0.008 g, 35.9% yield overall) was obtained from the filtrate.
$^1$H NMR (DMSO-$d_6$) δ: 2.37 (s, 3H); 2.38 (s,3H); 4.75 (d, J=6.6Hz, 2H); 6.17 (t, J=6.6Hz, 1H); 6.93 (s, 1H); 7.51 (d, J=8.7Hz, 1H); 7.55 (d, J=8.7 Hz, 1H).

Anal. Calcd. for $C_{16}H_{13}Cl_2N_3O_3$:
C, 52.58; H, 3.58; N, 11.47.
Found: C, 52.70; H, 3.35; N, 11.36.

EXAMPLE 20

Preparation of 3-[(4,7-Dichlorobenzoxazol-2-yl)methyl]amino-5-[1(R,S)-hydroxyethyl]-6-methylpyridin-2(1H)-one

Treatment of the product of Example 19 with sodium borohydride in ethanol results in reduction of the ketone to give the title compound.

EXAMPLE 21

Preparation of 3-[(5-Ethyl-2-methoxy-6-methylpyridin-3-yl)methyl]amino-5-[1(R,S)-hydroxyethyl]-6-methylpyridin-2(1H)-one

A mixture of 3-amino-5-(1-hydroxyethyl)-6-methylpyridin-2(1H)-one (0.034 g, 0.0002 mol), 5-ethyl-2-methoxy-6-methylpyridine-3-carboxaldehyde (0.036 g, 0.0002 mol), in toluene (5 mL) containing a drop of acetic acid was heated at reflux for 4 hours. Evaporation afforded a yellow solid (0.065 g) to which was added acetonitrile (3 mL), sodium cyanoborohydride (0.014 g), and acetic acid (0.013 mL). After stirring at room temperature for 6 hours chloroform and water, containing a few drops of acetic acid, were added. The chloroform phase was separated, and washed with saturated sodium bicarbonate, and brine, and then evaporated. The residue was dissolved in methanol with heating. An equal volume of acetonitrile was added and the solution left in the freezer for 20 hours. The product which gradually formed was filtered to give 0.015 g (22.6% yield) of the title compound as a colorless solid, mp 195-196°C.
$^1$H NMR ($CDCl_3$) δ: 1.12 (t,J=7.5Hz, 3H); 1.35 (d, J=6.3Hz, 3H); 2.21 (s, 3H); 2.40 (s, 3H); 2.51 (q,J=7.5Hz, 2H); 3.95 (s, 3H); 4.24 (s, 2H); 4.87 (q, J=6.3Hz, 1H); 5.12 (br s, 1H); 6.47 (s, 1H); 7.27 (s, 1H); 10.21 (br s, 1H).

Anal. Calcd. for $C_{18}H_{25}N_3O_3$:
C, 65.24; H, 7.60; N,12.68.
Found: C, 65.07; H, 7.47; N, 12.99.

EXAMPLE 22

Preparation of 5-Ethyl-3-[2-(4-fluorobenzoxazol-2-yl)ethyl]-6-hydroxymethylpyridin-2(1H)-one

Step A: Preparation of 2-Benzyloxy-6-benzyloxymethyl-5-ethyl-3-[2-(4-fluorobenzoxazol-2-yl)ethenyl]pyridine

Following the procedure described in Example 13, Step A, but substituting 2-chloromethyl-4-fluorobenzoxazole for 2-chloromethyl-4,7-difluorobenzoxazole, the desired title compound of Step A is prepared.

Step B: Preparation of 5-Ethyl-3-[2-(4-fluorobenzoxazol-2-yl)ethyl]-6-hydroxymethylpyridin-2(1H)-one

A solution of 2-benzyloxy-6-benzyloxymethyl-5-ethyl-3-[2-(4-fluorobenzoxazol-2-yl)ethenyl]pyridine (0.66 g, 1.33 mmol) in a mixture of THF (50 mL) and MeOH (75 mL) was hydrogenated over 5% Pd/C (100 mg) at room temp and atmospheric pressure for 18 h. The resultant mixture was filtered and concentrated. The residue was subjected to column chromatography on silica gel eluting with 3.5% MeOH in $CHCl_3$. Collection, concentration, and recrystallization from MeOH yielded 106 mg (25%) of analytically pure product, mp 144-5°C; $^1$H NMR (DMSO, 300MHz) δ 7.53 (dd, 1H, J =7.9, 0.6 Hz), 7.35 (m, 1H), 7.20 (m, 2H), 5.28 (t, 1H, J =5.5 Hz), 4.28 (d, 2H, J = 5.5 Hz), 3.21 (t, 2H, J = 7.5 Hz), 2.89 (t, 2H, J = 7.5 Hz), 2.28 (q, 2H, J = 7.3 Hz), 0.92 (t, 3H, J = 7.3 Hz).

Anal.Calcd for $C_{17}H_{17}FN_2O_3$:
C, 64.55; H, 5.42; N, 8.86.
Found: C, 64.41; H, 5.41; N, 8.70.

EXAMPLE 23

Preparation of 3-{N -[(5,6-Dimethyl-3-methoxypyridin-2-yl)methyl]amino}-5-ethyl-6-hydroxymethylpyridin-2(1H)-one

Step A: Preparation of 2-Methyl-3-oxobutanal, sodium salt

To a stirred solution of sodium methoxide (38.6 g, 0.71 mol) in absolute ethanol (90 mL) under an atmosphere of nitrogen, anhydrous diethyl ether (600 mL) was added. The resultant solution was cooled to 0°C, and a mixture of 2-butanone (51.2 g, 0.71 mol) and ethyl formate (57.2 g, 0.77 mol) was added over a period of 3.5 h. The resultant white slurry was stirred at room temperature overnight, filtered, washed with anhydrous diethyl ether (100 mL), and vacuum dried to yield regioselectively 43 g (49.7%) of the desired ketoaldehyde, sodium salt.

Step B: Preparation of 5,6-Dimethyl-3-nitro-2(1H)pyridinone

A mixture of 2-methyl-3-oxobutanal, sodium salt (43 g, 0.35 mol), nitroacetamide (35.6 g, 0.35 mol), aqueous piperidinium acetate (50 mL) [prepared from glacial acetic acid (42 mL), water (300 mL) and piperidine (72 mL)] in water (45 mL) was stirred at room temperature for 16 hours. The yellow slurry was cooled to 0°C and the precipitate was collected by filtration. The yellow solid obtained was then dried under vacuum overnight to yield 29 g (50%) of 5,6-dimethyl-3-nitro-(1H)-pyidinone.

Step C: Preparation of 2-Chloro-5,6-dimethyl-3-nitropyridine

A solution of 3-nitro-5,6-dimethyl-2(1H)-pyridinone (16.8 g, 0.1 mol) in phosphorus oxychloride (100 mL) was heated at 130°C for 6 h. Excess $POCl_3$ was distilled off under reduced pressure. The residue was poured into vigorously stirred ice-water. The off white solid precipitated was filtered and dissolved in methylene chloride. The resultant solution was washed with saturated aq. sodium bicarbonate, dried over anhydrous sodium sulfate, and passed through a plug of silica gel. Removal of solvent gave 17.2 g (92%) of 2-chloro-5,6-dimethyl-3-nitropyridine.

Step D: Preparation of 3-Amino-5,6-dimethylpyridine

A mixture of 2-chloro-5,6-dimethyl-3-nitropyridine (5 g, 26.8 mmol), triethylamine (10 mL), and 5% Pd/C (0.4 g) in methanol (200 mL) was shaken under an atmosphere of hydrogen (45 - 40 psi) for 16 h. The product mixture was filtered through a plug of Celite and concentrated under reduced pressure. The residue was then triturated with chloroform saturated with ammonia and the resultant slurry filtered through a plug of Celite. The filtrate was concentrated in vacuum to yield 3.2g (100%) of 3-amino-5,6-dimethylpyridine.

Step E: Preparation of 5,6-Dimethyl-3-hydroxypyridine

To a cold (0°C) solution of 3-amino-5,6-dimethylpyridine (4 g, 32.8 mmol) in 5% aq. sulfuric acid (100 mL), a solution of sodium nitrite (2.5 g, 36 mmol) in water (20 mL) was added dropwise. The resultant solution was stirred at 0°C for 30 min, transferred into an addition funnel maintained at 0°C with external cooling, and added dropwise into boiling 5% aq. sulfuric acid (70 mL) over a period of 30 min. The resultant solution was refluxed for additional 15 min, cooled to 0°C, neutralized with 40% aq. sodium hydroxide, saturated with addition of solid sodium chloride, and the product extracted into methylene chloride. The organic extract was dried over anhydrous sodium sulfate, filtered, and concentrated under reduced pressure to yield 3.5 g (86%) of crude 3-hydroxy-5,6-dimethylpyridine.

Step F: Preparation of 5,6-Dimethyl-2-hydroxymethyl-3-hydroxypyridine

To a solution of 3-hydroxy-5,6-dimethylpyridine (3.5 g, 28.4 mmol) and sodium hydroxide (1.2 g, 28.4 mmol) in water (12 mL) at 90°C, 38% aq. formaldehyde was added in four 2.5 mL aliquots in 90 min intervals. The resultant solution was heated at 90°C for additional 90 min, neutralized with acetic acid, and concentrated under reduced pressure. The residue was triturated with 10% methanol in chloroform saturated with ammonia, and filtered through a plug of Celite. The filtrate was concentrated and the residue subjected to column chromatography on silica gel eluted with 10% methanol in chloroform. Collection and concentration of appropriate fractions gave 2.1 g (48%) of 2-hydroxymethyl-3-hydroxy-5,6-dimethylpyridine.

Step G: Preparation of 5,6-Dimethyl-2-hydroxymethyl-3-methoxypyridine

To a solution of 2-hydroxymethyl-3-hydroxy-5,6-dimethylpyridine (1.16 g, 7.6 mmol) and sodium hydride (0.18 g, 7.6 mmol) in anhydrous dimethylformamide (20 mL) at 0°C, methyl iodide (1.08 g, 7.6 mmol) was added and stirred at 0°C for 30 min, and at room temp for 1 h. The resultant solution was concentrated under reduced pressure, and the residue subjected to column chromatography on silica gel eluted with 2% methanol in chloroform. Collection and concentration of appropriate fractions gave 0.7 g (55%) of 2-hydroxymethyl-3-methoxy-5,6-dimethylpyridine.

Step H: Preparation of 5,6-Dimethyl-3-methoxy-2-picolinaldehyde

A slurry of 2-hydroxymethyl-3-methoxy-5,6-dimethylpyridine (0.7 g, 4.2 mmol) and activated manganese (IV) oxide (8 g) in methylene chloride (40 mL) was stirred at rt overnight. The resultant mixture was filtered through a plug of Celite and the filtrate concentrated to give 0.4 g (58%) of 5,6-dimethyl-3-methoxy-2-picolinaldehyde.

Step I: Preparation of 3-{N -[(5,6-Dimethyl-3-methoxypyridin-2-yl)methyl]amino}-5-ethyl-6-hydroxymethylpyridin-2(1H)-one

A solution of 3-amino-5-ethyl-6-hydroxymethylpyridin-2(1H)-one (100 mg, 0.59 mmol), 5,6-dimethyl-3-methoxy-2-picolinaldehyde (98 mg, 0.59 mmol), and acetic acid (20 μL) in methanol (5 mL) was stirred at room temp for 17 h. The resultant yellow solution was treated with sodium borohydride until the solution became colorless. The product solution was then concentrated onto silica gel (2 g), and subjected to column chromatography on silica gel eluting with 3.5-5% methanol in chloroform gradient. Collection, concentration of appropriate fraction, and recrystallization from a mixture of acetonitrile-methanol-water yielded 38 mg (20%) of the title compound, mp 202-5°C;
$^1$H NMR (DMSO, 300MHz) δ 7.25 (s,1H), 6.23 (s, 1H), 5.99 (t, 1H, J =5 Hz), 4.90 (t, 1H, J = 5 Hz), 4.22 (d, 2H, J = 5 Hz), 4.15 (d, 2H, J = 5 Hz), 3.82 (s, 3H), 2.37 (m, 5H), 2.25 (s, 3H), 1.07 (t, 3H, J = 7.3 Hz).

Anal.Calcd for $C_{17}H_{23}N_3O_3$:
C, 64.33; H, 7.30; N, 13.24.
Found: C, 64.22; H, 6.93; N, 13.17.

EXAMPLE 24

Preparation of 5-Ethyl-6-hydroxymethyl-3-{N -[(3-methoxy-5,6,7,8-tetrahydroquinolin-2-yl)methyl]amino}pyridin-2(1H)-one

Following procedures described in Example 23 and starting with cyclohexanone, 5-Ethyl-6-hydroxymethyl-3-{N -[(3-methoxy-5,6,7,8-tetrahydroquinolin-2-yl)methyl]amino}pyridin-2(1H)-one, mp 194-5°C (acetonitrile) was prepared.
$^1$H NMR ($CDCl_3$, 300MHz) δ 7.26 (s,1H), 6.42 (s, 1H), 4.64 (br s, 2H), 4.56 (br s, 2H), 3.97 (s, 3H), 3.05 (t, 2H, J = 5 Hz), 2.83 (t, 2H, J = 5 Hz), 2.39 (q, 2H, J = 7.3 Hz), 1.82 (m, 4H), 1.07 (t, 3H, J = 7.3 Hz).

Anal.Calcd for $C_{19}H_{25}N_3O_3$ . 0.3 $H_2O$:
C, 65.42; H, 7.40; N, 12.05.
Found: C, 65.44; H, 7.11; N, 12.02.

Example 25

Preparation of 5-Ethyl-3-{N -[(6-ethyl-3-methoxy-5-methylpyridin-2-yl)methyl]amino}-6-hydroxymethylpyridin-2(1H)-one

Following procedures described in Example 23 and starting with 2-pentanone, 5-Ethyl-3-{N -[(6-ethyl-3-methoxy-5-methylpyridin-2-yl)methyl]amino}-6-hydroxymethylpyridin-2(1H)-one, mp 193-5°C (acetonitrile) was prepared.
$^1$H NMR ($CDCl_3$, 300MHz) δ 7.08 (s,1H), 6.46 (s, 1H), 4.56 (br s, 2H), 4.46 (br s, 2H), 3.90 (s, 3H), 2.85 (q, 2H, J = 7.5 Hz), 2.40 (q, 2H, J = 7.5 Hz), 2.34 (s, 3H), 1.29 (t, 3H, J = 7.5 Hz), 1.12 (t, 3H, J = 7.5 Hz).

Anal.Calcd for $C_{18}H_{25}N_3O_3$ . 0.25 $H_2O$:
C, 64.36; H, 7.65; N, 12.51.
Found: C, 64.57; H, 7.35; N, 12.12.

EXAMPLE 26

Preparation of 3-{N-[(5,6-Dimethyl-2-methoxypyridin-3-yl)methyl]amino}-5-ethyl-6-hydroxymethylpyridin-2(1H)-one

Following the procedures described in Example 11 and starting with 5,6-dimethyl-2-methoxynicotinaldehyde, the title compound is obtained.

EXAMPLE 27

Preparation of 5-Ethyl-6-hydroxymethyl-3-{N-[(2-methoxy-5,6,7,8-tetrahydroquinolin-3-yl)methyl]amino}pyridin-2(1H)-one

Following the procedures described in Example 11 and starting with 2-methoxy-5,6,7,8-tetrahydroquinoline-3-carboxaldehyde, the title compound is obtained.

EXAMPLE 28

Preparation of 3-{N-[(2-Methoxy-5,6-trimethylenepyridin-3-yl)methyl]amino}-5-ethyl-6-hydroxymethylpyridin-2(1H)-one

Following the procedures described in Example 11 and starting with 2-methoxy-5,6-trimethylenenicotinaldehyde, the title compound is obtained.

EXAMPLE 29

Preparation of 3-{N-[(5-Ethyl-2-methoxypyridin-3-yl)methyl]amino}-5-ethyl-6-hydroxymethylpyridin-2(1H)-one

Following the procedures of Example 11 and starting with 5-ethyl-2-methoxynicotinaldehyde, the title compound is obtained.

EXAMPLE 30

Preparation of 5-Ethyl-6-hydroxymethyl-3-{N-[(2-methoxyquinolin-3-yl)methyl]amino}pyridin-2(1H)-one

Following the procedures of Example 11 and starting with 2-methoxyquinoline-3-carboxaldehyde, the title compound is obtained.

EXAMPLE 31

Preparation of 3-{N-[(3-Methoxy-5,6-trimethylenepyridin-2-yl)methyl]amino}-5-ethyl-6-hydroxymethylpyridin-2(1H)-one

Following the procedures of Example 11 and starting with 3-methoxy-5,6-trimethylene-2-picolinaldehyde, the title compound is obtained.

EXAMPLE 32

Preparation of 3-{N-[(3-Methoxy-6-ethylpyridin-2-yl)methyl]amino}-5-ethyl-6-hydroxymethylpyridin-2(1H)-one

Following the procedures of Example 11 and starting with 3-methoxy-6-ethyl-2-picolinaldehyde, the title compound is obtained.

EXAMPLE 33

Preparation of 5-Ethyl-6-hydroxymethyl-3-{N-[(3-methoxyquinolin-2-yl)methyl]amino}pyridin-2(1H)-one

Following the procedures of Example 11 and starting with 3-methoxyquinoline-2-carboxaldehyde, the title compound is obtained.

EXAMPLE

Reverse Transcriptase Assay

The assay measures the incorporation of tritiated deoxyguanosine monophosphate by recombinant HIV reverse transcriptase (HIV $RT_R$) (or other RT) into acid-precipitable cDNA at the Km values of dGTP and poly r(C)•oligo $d(G)_{12-18}$. The inhibitors of the present invention inhibit this incorporation.

Thirty uL of a reaction mixture containing equal volumes of: 500 mM Tris•HCl (pH 8.2), 300 mM $MgCl_2$, 1200 mM KCl, 10 mM DTT, 400 μg/mL poly r(c)•oligo d(G) [prepared by dissolving 1.5 mg (25 U) poly r(C)•oligo d(G) in 1.5 ml sterile distilled $H_2O$ and diluting to 400 μg/ml], 0.1 μCi/μl [$^3$H] dGTP, 160 μM dGTP, was added to 10 μl sterile distilled $H_2O$, 2.5 μl of potential inhibitor and 10 μL of 5 nM purified HIV $RT_R$ in tubes. The mixture was incubated at 37°C for 45 minutes.

After incubation is complete, the tubes were cooled in ice for 5 minutes. Ice-cold 13% TCA containing 10

mM $NaPP_i$ (200 µl) are added and the mixture incubated on ice for 30 minutes. The precipitated cDNA is removed by filtration using presoaked glass filters [TCA, $NaPP_i$]. The precipitate is then washed with 1N HCl, 10 mM $NaPP_i$.

The filter discs are then counted in a scintillation counter.

Under these conditions [dGTP] and poly r(C)•oligo $d(G)_{12-18}$ each are approximately equal to the appropriate Km value. Approximately 5-6,000 cpm of [$^3$H] GMP are incorporated into acid-precipitable material. The RT reaction is concentration- and time-dependent. DMSO (up to 5%) does not affect enzyme activity. Calculated $IC_{50}$ values for the lead compounds of this invention were approximately 94nM and (more than or equal to) 300 µM for L-697,914 and L-697,913, respectively. Other preferred compounds gave $IC_{50}$ values of between about 5nM and about 100nM, the most preferred having $IC_{50}$ values between about 11nM and 14nM.

While the foregoing specification teaches the principles of the present invention, with examples provided for the purpose of illustration, it will be understood that the practice of the invention encompasses all of the usual variations, adaptations, and modifications, as come within the scope of the following claims and its equivalents.

## Claims

1. A compound of the formula

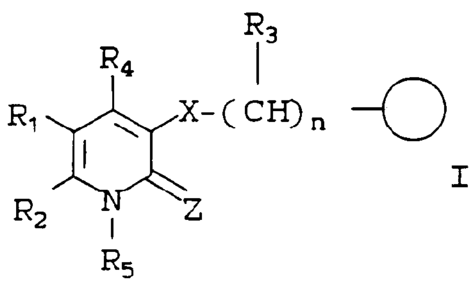

I

wherein

$R_1$ or $R_2$ or both are substituted at least once with OH;

X is 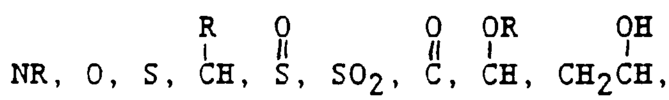

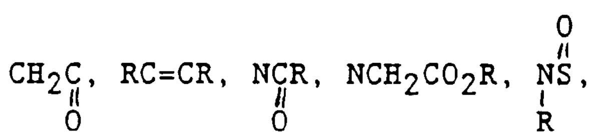

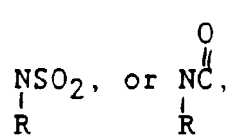

where

R is H, $C_{1-8}$ alkyl, $C_{1-8}$ alkenyl or $C_{3-8}$ cycloalkyl;

Z is O, S or $NR_x$ when $R_x$ is H or $C_{1-8}$ alkyl;

n is 0-4;

$R_1$, $R_2$ and $R_4$ are the same or different and are independently

(i) H;
(ii) $C_{1-8}$ alkyl, $C_{1-8}$ alkenyl, $C_{3-8}$cycloalkyl; any of which is unsubstituted or substituted with one or two of $C_{1-3}$ alkoxy, $C_{1-4}$ alkylamino, di($C_{1-4}$ alkyl)amino, $C_{1-3}$ alkylthio, hydroxy, amino, oxo, carbonyl, aminocarbonyl, or oximido, or one to five of halo;
(iii) $C_{1-5}$ alkylthio;
(iv) $C_{1-5}$ alkylsulfinyl;
(v) $C_{1-5}$ alkylsulfonyl;
(vi) $C_{1-5}$ alkoxy;
(vii) $C_{1-5}$ alkoxycarbonyl;
(viii) cyano; or
(ix) aryl;

or, $R_1$ and $R_4$ may together form a cycloalkyl ring containing 5-7 members;
or, $R_1$ and $R_2$ may together form a cycloalkyl ring containing 5-7 members;
and $R_3$ or $R_5$ are the same or different and are independently

(i) H;
(ii) $C_{1-8}$ alkyl;
(iii) $C_{1-8}$ alkenyl;
(iv) $C_{3-8}$ cycloalkyl;

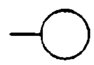

is aryl or heterocycle, each unsubstituted or substituted with one or more of

(i) $C_{1-6}$ alkyl unsubstituted or substituted with one or more of A, wherein A is halo, hydroxy, hydroxy-$C_{1-4}$ alkyl, amino, $C_{1-6}$ alkylamino, di($C_{1-6}$ alkyl)amino or aryl;
(ii) $C_{1-6}$ alkenyl unsubstituted or substituted with one or more of A;
(iii) $C_{3-6}$ cycloalkyl unsubstituted or substituted with one or more of A;
(iv) $C_{1-6}$ alkoxy unsubstituted or substituted with one or more of A;
(v) aryl;
(vi) amino;
(vii) $C_{1-6}$ alkylamino;
(viii) di($C_{1-6}$ alkyl)amino;
(ix) amino-$C_{1-8}$ alkyl;
(x) $C_{1-8}$ alkyl-amino-$C_{1-8}$ alkyl;
(xi) di-($C_{1-6}$ alkyl)amino $C_{1-8}$ alkyl;
(xii) $C_{1-6}$ alkoxycarbonyl;
(xiii) aminocarbonyl;
(xiv) $C_{1-6}$ alkyl aminocarbonyl;
(xv) di($C_{1-6}$ alkyl)aminocarbonyl;
(xvi) $C_{1-6}$ alkylthio;
(xvii) $C_{1-6}$ alkylsulfinyl;
(xviii) $C_{1-6}$ alkylsulfonyl;
(xix) hydroxy;
(xx) halo;
(xxi) CN; or
(xxii) $NO_2$;

with the proviso that heterocycle is not phthalimide;
or pharmaceutically acceptable salt or ester thereof.

**2.** A compound according to Claim 1 of the formula

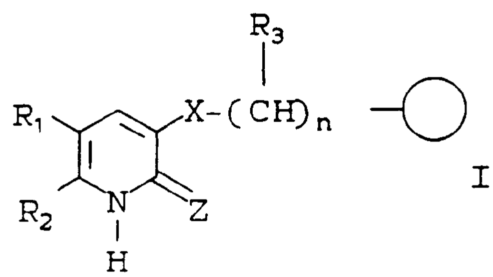

II

wherein:
X is NH or $CH_2$;
Z is O or S;
n is 1-4;
$R_1$ is
(i) $C_{1-8}$ alkyl, unsubstituted or substituted with one or two of $C_{1-3}$alkoxy, halo, oxo, hydroxyl, $C_{1-4}$-alkylamino, $C_{1-4}$-dialkylamino, or $C_{1-3}$ alkylthio;
(ii) $C_{1-3}$ alkylthio;
(iii) $C_{1-3}$ alkoxy; or
$R_2$ is H, methyl or ethyl, unsubstituted or substituted with one or two of methoxy, hydroxyl, methylamino, dimethylamino or methylthio;
provided that one of $R_1$ or $R_2$, or both, is substituted at least once with hydroxyl;
$R_3$ is H or $C_{1-8}$ alkyl;

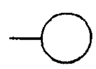

is aryl or heterocycle, each unsubstituted or substituted with one or more of
(a) $C_{1-6}$ alkyl,
(b) $C_{1-6}$ alkoxy unsubstituted or substituted with hydroxy-$C_{1-4}$ alkyl,
(c) amino,
(d) $C_{1-6}$ alkyl amino,
(e) di($C_{1-6}$ alkyl)amino,
(f) amino- $C_{1-8}$ alkyl
(g) $C_{1-8}$ alkyl-amino-$C_{1-3}$ alkyl,
(h) di-($C_{1-6}$ alkyl)amino $C_{1-8}$ alkyl,
(i) hydroxy, or
(j) halo,
with the proviso that heterocycle is not phthalimide, or pharmaceutically acceptable salt or ester thereof.

3. A compound according to Claim 2, of the formula

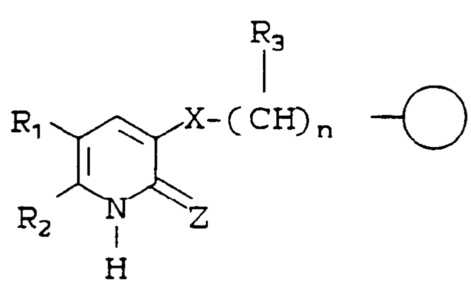

wherein:

X is NH;
Z is O or S;
n is 1-4;
$R_1$ is
(i) $C_{1-8}$ alkyl, unsubstituted or substituted with one or two of $C_{1-3}$alkoxy, halo, hydroxyl, $C_{1-4}$alkylamino, $C_{1-4}$-dialkylamino, or $C_{1-3}$ alkylthio;
(ii) $C_{1-3}$ alkylthio;
(iii) $C_{1-3}$ alkoxy; or
( iv) halo;
$R_2$ is H, methyl or ethyl, unsubstituted or substituted with one or two of methoxy, hydroxyl, methylamino, dimethylamino or methylthio;
provided that one of $R_1$ or $R_2$, or both, is substituted at least once with hydroxyl;
$R_3$ is H or $C_{1-8}$ alkyl;

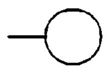

is aryl or heterocycle, each unsubstituted or substituted with one or more of
(a) $C_{1-6}$ alkyl,
(b) $C_{1-6}$ alkoxy unsubstituted or substituted with hydroxy-$C_{1-4}$ alkyl,
(c) amino,
(d) $C_{1-6}$ alkyl amino,
(e) di($C_{1-6}$ alkyl)amino,
(f) amino- $C_{1-8}$ alkyl,
(g) $C_{1-8}$ alkyl-amino-$C_{1-3}$ alkyl,
(h) di-($C_{1-6}$ alkyl)amino $C_{1-8}$ alkyl,
(i) hydroxyl, or
(j) halogen,
with the proviso that heterocycle is not phthalimide, or pharmaceutically acceptable salt or ester thereof.

**4.** A compound of claim 3, wherein:
$R_1$ is $C_{1-4}$ alkyl, unsubstituted or substituted with one or more of hydroxyl;
$R_2$ is H, $CH_3$ or $CH_2OH$;
provided that one of $R_1$ or $R_2$, or both, is substituted at least once with hydroxyl;
aryl is
(a) naphthyl, unsubstituted or substituted with one or more of $C_{1-6}$ alkyl, $C_{1-6}$ alkoxy, halogen or hydroxyl;
(b) phenyl, unsubstituted or substituted with one or more of $C_{1-6}$ alkyl, $C_{1-6}$ alkoxy, halogen or hydroxyl; or
(c) biphenyl.

**5.** A compound of Claim 2 wherein heterocycle is thienyl, oxazolyl, thiazolyl, triazolyl, pyridyl unsubstituted or substituted with one or more of $C_{1-2}$alkoxy or $C_{1-3}$alkyl or hydroxyalkyl, pyridinonyl, pyrazinyl, benzothienyl, quinolinyl unsubstituted or substituted with $C_{1-2}$ alkoxy, 5,6,7,8-tetrahydroquinolinyl unsubstituted or substituted with $C_{1-2}$ alkoxy, isoquinolinyl unsubstituted or substituted with $C_{1-2}$ alkoxy, benzimidazolyl, benzofuranyl, benzothiazolyl, benzoxazolyl unsubstituted or substituted with one or more of $C_{1-6}$ alkyl or halogen or hydroxyl, 4,5,6,7-tetrahydrobenzoxazolyl, naphth [3,2-d] oxazolyl, oxazolo [4,5-b] pyridyl, chromone, or benzopyrimidinone.

**6.** A compound of Claim 5 wherein heterocycle is 2-oxazolyl, 2-pyridyl, 3-pyridyl unsubstituted or substituted with one or more of $C_{1-2}$alkoxy or $C_{1-3}$alkyl, 2-benzothienyl, 2-quinolinyl, 2-(5,6,7,8-tetrahydroquinolinyl) unsubstituted or substituted with $C_{1-2}$ alkoxy 2-benzimidazolyl, 2-benzofuranyl, 2-benzothiazolyl, 2-oxazolo [4,5-b] pyridyl, or 2-benzoxazolyl unsubstituted or substituted with one or more of methyl, halogen or hydroxyl.

**7.** A compound of Claim 6 wherein heterocycle is 2-methoxy-5,6-dimethyl-3-pyridyl, 2-methoxy-5-ethyl-6-methyl-3-pyridyl, 3-methoxy-5,6-dimethyl-2-pyridyl, 3-methoxy-5-methyl-6-ethyl-2-pyridyl, 2-benzoxazolyl, 7-methyl-2-benzoxazolyl, 4,7-dimethyl-2-benzoxazolyl, 7-chloro-2-benzoxazolyl, 4,7-dichloro-2-benzoxazolyl, 4-fluoro-2-benzoxazolyl, or 4,7-difluoro-2-benzoxazolyl.

8. A compound of claim 7 wherein Heterocycle is 2-methoxy-5-ethyl-6-methyl-3-pyridyl, 2-benzoxazolyl, 7-methyl-2-benzoxazolyl, 4,7-dimethyl-2-benzoxazolyl, 7-chloro-2-benzoxazolyl, or 4,7-dichloro-2-benzoxazolyl.

9. A compound, which is
3-[2-(4,7-dichlorobenzoxazol-2-yl)ethyl]-5-ethyl-6-hydroxymethylpyridin-2(1H)-one,
3-[(4,7-dichlorobenzoxazol-2-yl)methylamino]-5-ethyl-6-hydroxymethylpyridin-2(1H)-one,
3-{N-[(2-methoxy-5-ethyl-6-methylpyridin-3-yl)methyl]amino}-5-ethyl-6-hydroxymethylpyridin-2(1H)-one,
3-[2-(4,7-Dichlorobenzoxazol-2-yl)ethyl]-5-ethyl-6-hydroxymethylpyridin-2(1H)-thione,
3-[(4,7-Dichlorobenzoxazol-2-yl)methyl]amino-5-[1(R,S)-hydroxyethyl]-6-methylpyridin-2(1H)-one,
3-[(4,7-Dichlorobenzoxazol-2-yl)methyl]amino-5-(1-oxoethyl)-6-methylpyridin-2(1H)-one,
3-[(4,7-Dichlorobenzoxazol-2-yl)methyl]amino-5-[1-(S,R)-hydroxyethyl)-6-methylpyridin-2(1H)-one,
3-[(5-Ethyl-2-methoxy-6-methylpyridin-3-yl)methyl]amino-5-[1(R,S)-hydroxyethyl]-6-methylpyridin-2(1H)-one,
5-Ethyl-3-[2-(4-fluorobenzoxazol-2-yl)ethyl]-6-hydroxymethylpyridin-2(1H)-one,
3-{N-[(5,6-Dimethyl-3-methoxypyridin-2-yl)methyl]amino}-5-ethyl-6-hydroxymethylpyridin-2(1H)-one,
5-Ethyl-6-hydroxymethyl-3-(N-[(3-methoxy-5,6,7,8-tetrahydroquinolin-2-yl)methyl]amino}pyridin-2(1H)-one,
5-Ethyl-3-{N-[(6-ethyl-3-methoxy-5-methylpyridin-2-yl)methyl]amino}-6-hydroxymethylpyridin-2(1H)-one,
3-{N-[(5,6-dimethyl-2-methoxypyridin-3-yl)methyl]amino}-5-ethyl-6-hydroxymethylpyridin-2(1H)-one,
5-Ethyl-6-hydroxymethyl-3-{N-[(2-methoxy-5,6,7,8-tetrahydroquinolin-3-yl)methyl]amino}pyridin-2(1H)-one,
3-{N-[(2-methoxy-5,6-trimethylene-pyridin-3-yl)methyl]amino}-5-ethyl-6-hydroxymethylpyridin-2(1H)-one,
3-{N-[(6-methoxy-indan-5-yl)methyl]amino}-5-ethyl-6-hydroxymethylpyridin-2(1H)-one,
3-{N-[(5-ethyl-2-methoxypyridin-3-yl)methyl]amino}-5-ethyl-6-hydroxymethylpyridin-2(1H)-one,
5-Ethyl-6-hydroxymethyl-3-{N-[(2-methoxyquinolin-3-yl)methyl]amino}pyridin-2(1H)-one,
3-{N-[(3-methoxy-5,6-trimethylene-pyridin-2-yl)methyl]amino}-5-ethyl-6-hydroxymethylpyridin-2(1H)-one,
3-{N-[(3-methoxy-5-ethylpyridin-2-yl)methyl]amino}-5-ethyl-6-hydroxymethylpyridin-2(1H)-one,
3-[N-(5-ethyl-2-methoxybenzyl)amino]-5-ethyl-6-hydroxymethylpyridin-2(1H)-one,
3-[2-(4,7-difluorobenzoxazol-2-yl)ethyl]-5-ethyl-6-hydroxymethylpyridin-2(1H)-one, or
3-[N-(4,5-dimethyl-2-methoxybenzyl)amino]-5-ethyl-6-hydroxymethylpyridin-2(1H)-one,
or pharmaceutically acceptable salt or ester thereof.

10. A compound of the formula:

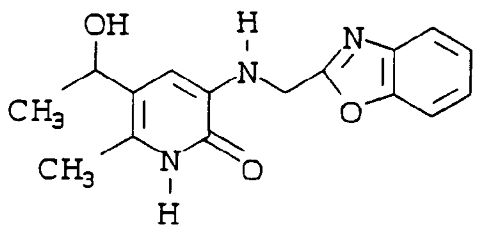

or pharmaceutically acceptable ester thereof.

11. A compound of the formula:

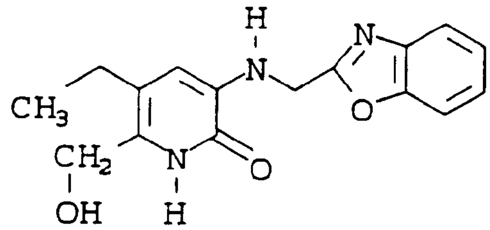

or pharmaceutically acceptable ester thereof.

**12.** A compound of the formula:

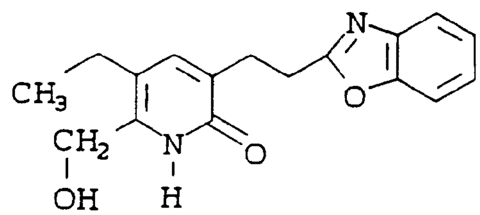

or pharmaceutically acceptable ester thereof.

**13.** A compound of the formula:

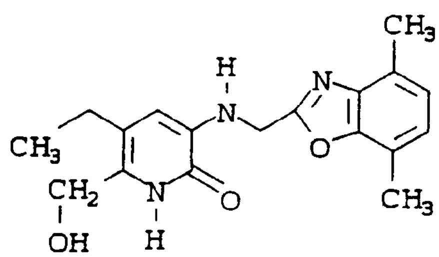

or pharmaceutically acceptable ester thereof.

**14.** A compound of the formula:

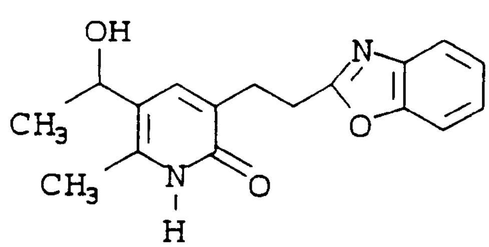

or pharmaceutically acceptable ester thereof.

15. A compound of the formula:

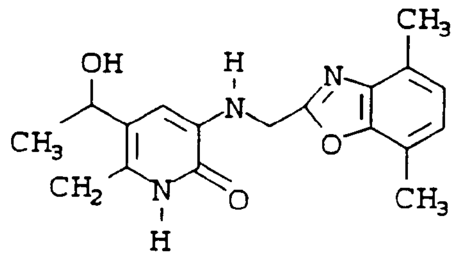

or pharmaceutically acceptable ester thereof.

16. A compound of the formula

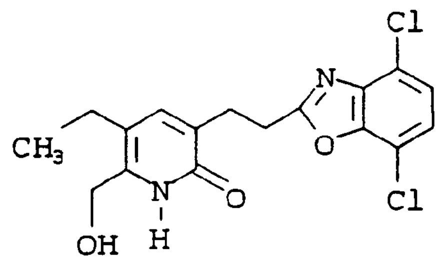

or pharmaceutically acceptable ester thereof.

17. A compound of the formula

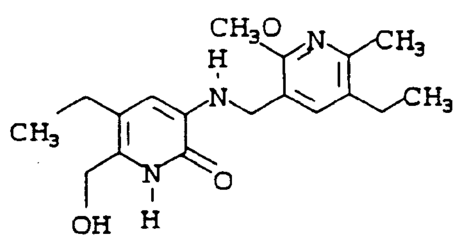

or pharmaceutically acceptable ester thereof.

18. A compound of the formula

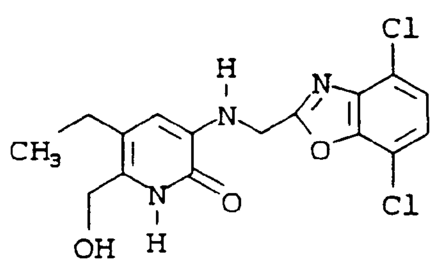

or pharmaceutically acceptable ester thereof.

**19.** A compound of the formula

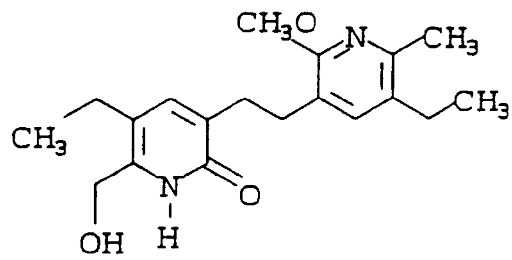

or pharmaceutically acceptable ester thereof.

**20.** A compound of the formula

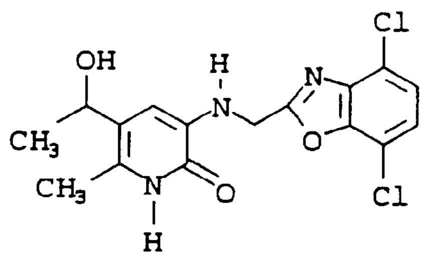

or pharmaceutically acceptable ester thereof.

**21.** A compound of the formula

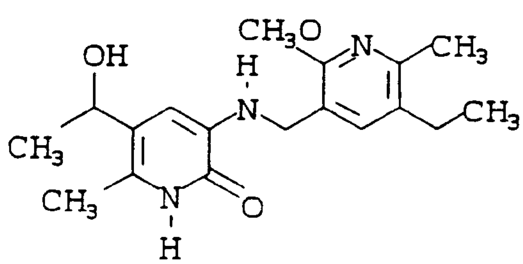

or pharmaceutically acceptable ester thereof.

**22.** A method of preparing the compounds of Claim 10 or 11, comprising the steps of:
(a) providing a quantity of 3-[(2-benzoxazolyl-methyl)amino]-5-ethyl-6-methyl-2-(1H)-pyridinone;
(b) incubating the sample of step (a) with culture MA6559(ATCC 53771); and
(c) isolating the compound of Claim 10 or 11.

**23.** The use of a compound as claimed in any of claims 1-21, for the preparation of a medicament useful for inhibiting HIV reverse transcriptase.

**24.** The use of a compound as in any of claims 1-21, for the preparation of a medicament useful for preventing infection of HIV, or of treating infection by HIV or of treating AIDS or ARC.

**25.** A pharmaceutical composition useful for inhibiting HIV reverse transcriptase, comprising an effective amount of a compound as in any of claims 1-21 and a pharmaceutically acceptable carrier.

**26.** A pharmaceutical composition useful for preventing or treating infection of HIV or for treating AIDS or ARC, comprising an effective amount of a compound as in any of claims 1-21, and a pharmaceutically acceptable carrier.

**27.** A process for synthesizing a compound of formula III,

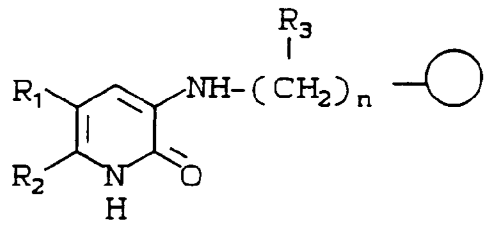

III

wherein
n is 1-4;
$R_1$ is
(i) $C_{1-8}$ alkyl, unsubstituted or substituted with one or two of $C_{1-3}$alkoxy, halo, hydroxyl, $C_{1-4}$alkylamino, $C_{1-4}$-di-(alkyl)amino, or $C_{1-3}$ alkylthio;
(ii) $C_{1-3}$ alkylthio;
(iii) $C_{1-3}$ alkoxy; or
( iv) halo;
$R_2$ is H, methyl or ethyl, unsubstituted or substituted with one or two of methoxy, hydroxyl, methylamino, dimethylamino or methylthio; provided that one of $R_1$ or $R_2$, or both, is substituted at least once with hydroxyl;
$R_3$ is H or $C_{1-8}$ alkyl;

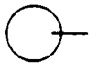

is aryl or heterocyle, each unsubstituted or substituted with one or more of
(a) $C_{1-6}$ alkyl,
(b) $C_{1-6}$ alkoxy unsubstituted or substituted with hydroxy-$C_{1-4}$ alkyl,
(c) amino,
(d) $C_{1-6}$ alkyl amino,
(e) di($C_{1-6}$ alkyl)amino,
(f) amino- $C_{1-8}$ alkyl,
(g) $C_{1-8}$ alkyl-amino-$C_{1-3}$ alkyl,
(h) di-($C_{1-6}$ alkyl)amino $C_{1-8}$ alkyl,
(i) hydroxyl, or
(j) halogen,
with the proviso that heterocycle is not phthalimide;
comprising the step of condensing

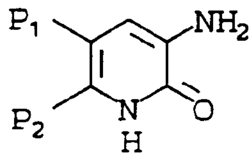

1

and

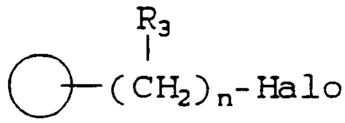

2

by alkylation in a process yielding a compound of Formula III.

**28.** A process for synthesizing a compound of Formula III,

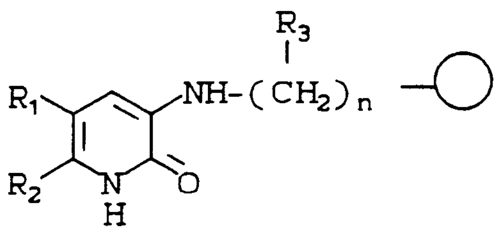

III

wherein
n is 1-4;
$R_1$ is
(i) $C_{1-8}$ alkyl, unsubstituted or substituted with one or two of $C_{1-3}$alkoxy, halo, hydroxyl, $C_{1-4}$alkylamino, $C_{1-4}$-di-(alkyl)amino, or $C_{1-3}$ alkylthio;
(ii) $C_{1-3}$ alkylthio;
(iii) $C_{1-3}$ alkoxy; or
( iv) halo;
$R_2$ is H, methyl or ethyl, unsubstituted or substituted with one or two of methoxy, hydroxyl, methylamino, dimethylamino or methylthio; provided that one of $R_1$ or $R_2$, or both, is substituted at least once with hydroxyl;
$R_3$ is H or $C_{1-8}$ alkyl;

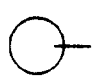

is aryl or heterocycle, each unsubstituted or substituted with one or more of
(a) $C_{1-6}$ alkyl,
(b) $C_{1-6}$ alkoxy unsubstituted or substituted with hydroxy-$C_{1-4}$ alkyl,

(c) amino,
(d) $C_{1-6}$ alkyl amino,
(e) di($C_{1-6}$ alkyl)amino,
(f) amino- $C_{1-8}$ alkyl,
(g) $C_{1-8}$ alkyl-amino-$C_{1-8}$ alkyl,
(h) di-($C_{1-6}$ alkyl)amino $C_{1-8}$ alkyl,
(i) hydroxyl, or
(j) halogen,

with the proviso that heterocycle is not phthalimide;
comprising the step of condensing

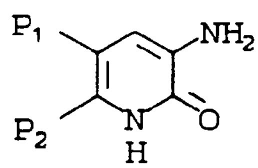

1

and

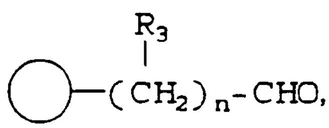

3

by reductive alkylation in a process yielding a compound of Formula III.

**29.** A process for synthesizing a compound of Formula IV,

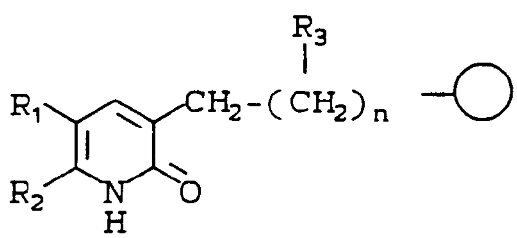

IV

wherein
n is 1-4;
$R_1$ is

(i) $C_{1-8}$ alkyl, unsubstituted or substituted with one or two of $C_{1-3}$alkoxy, halo, hydroxyl, $C_{1-4}$alkylamino, $C_{1-4}$-di-(alkyl)amino, or $C_{1-3}$ alkylthio;
(ii) $C_{1-3}$ alkylthio;
(iii) $C_{1-3}$ alkoxy; or
( iv) halo;

$R_2$ is H, methyl or ethyl, unsubstituted or substituted with one or two of methoxy, hydroxyl, methylamino, dimethylamino or methylthio;
provided that $R_1$ or $R_2$, or both, is substituted at least once with hydroxyl;
$R_3$ is H or $C_{1-8}$ alkyl;

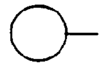

is aryl or heterocycle, each unsubstituted or substituted with one or more of

(a)$C_{1-6}$ alkyl,
(b) $C_{1-6}$ alkoxy unsubstituted or substituted with hydroxy-$C_{1-4}$ alkyl,
(c) amino,
(d) $C_{1-6}$ alkyl amino,
(e) di($C_{1-6}$ alkyl)amino,
(f) amino- $C_{1-8}$ alkyl,
(g) $C_{1-8}$ alkyl-amino-$C_{1-8}$ alkyl,
(h) di-($C_{1-6}$ alkyl)amino $C_{1-8}$ alkyl,
(i) hydroxyl, or
(j) halogen,

with the proviso that heterocycle is not phthalimide;
comprising conversion of the intermediate

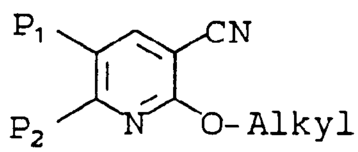

to the intermediate

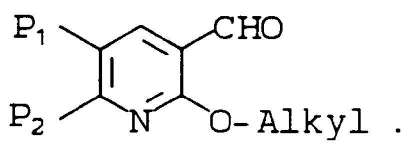

**30.** A process for synthesizing a compound of Formula IV,

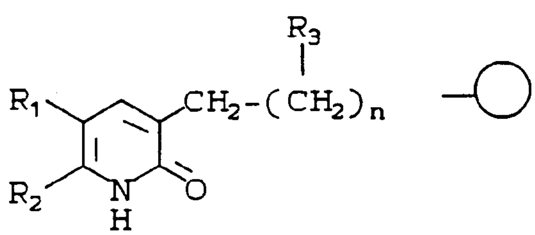

IV

wherein
n is 1-4;
$R_1$ is

(i) $C_{1-8}$ alkyl, unsubstituted or substituted with one or two of $C_{1-3}$alkoxy, halo, hydroxyl, $C_{1-4}$alkylamino, $C_{1-4}$-di-(alkyl)amino, or $C_{1-3}$ alkylthio;
(ii) $C_{1-3}$ alkylthio;
(iii) $C_{1-3}$ alkoxy; or
( iv) halo;

$R_2$ is H, methyl or ethyl, unsubstituted or substituted with one or two of methoxy, amino, methylamino,

dimethylamino or methylthio;
provided that one of $R_1$ or $R_2$, or both, is substituted at least once with hydroxyl;
$R_3$ is H or $C_{1-8}$ alkyl;

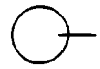

is aryl or heterocycle, each unsubstituted or substituted with one or more of

(a) $C_{1-6}$ alkyl,
(b) $C_{1-6}$ alkoxy unsubstituted or substituted with hydroxy-$C_{1-4}$ alkyl,
(c) amino,
(d) $C_{1-6}$ alkyl amino,
(e) di($C_{1-6}$ alkyl)amino,
(f) amino- $C_{1-8}$ alkyl,
(g) $C_{1-8}$ alkyl-amino-$C_{1-8}$ alkyl,
(h) di-($C_{1-6}$ alkyl)amino $C_{1-8}$ alkyl,
(i) hydroxyl or,
(j) halogen,

with the proviso that heterocycle is not phthalimide;
comprising conversion of the intermediate

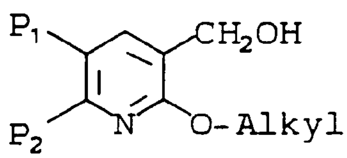

to the intermediate

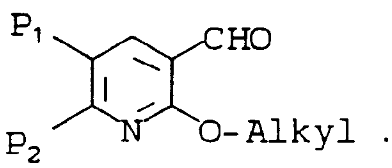

**31.** A process for synthesizing a compound of formula V,

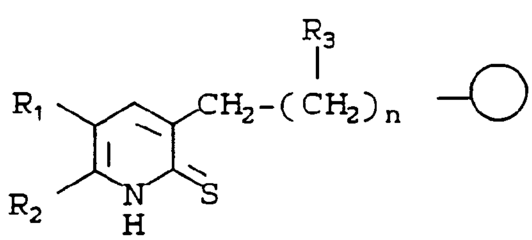

V

wherein
n is 1-4;
$R_1$ is

(i) $C_{1-8}$ alkyl, unsubstituted or substituted with one or two of $C_{1-3}$alkoxy, halo, hydroxyl, $C_{1-4}$alkylamino, $C_{1-4}$-di-(alkyl)amino, or $C_{1-3}$ alkylthio;

(ii) $C_{1-3}$ alkylthio;
(iii) $C_{1-3}$ alkoxy; or
( iv) halo;
$R_2$ is H, methyl or ethyl, unsubstituted or substituted with one or two of methoxy, hydroxyl, methylamino, dimethylamino or methylthio;
provided that one of $R_1$ or $R_2$, or both, is substituted at least once with hydroxyl;
$R_3$ is H or $C_{1-8}$ alkyl;

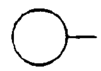

is aryl or heterocycle, each unsubstituted or substituted with one or more of
(a) $C_{1-6}$ alkyl,
(b) $C_{1-6}$ alkoxy unsubstituted or substituted with hydroxy-$C_{1-4}$ alkyl,
(c) amino,
(d) $C_{1-6}$ alkyl amino,
(e) di($C_{1-6}$ alkyl)amino,
(f) amino- $C_{1-8}$ alkyl,
(g) $C_{1-8}$ alkyl-amino-$C_{1-3}$ alkyl,
(h) di-($C_{1-6}$ alkyl)amino $C_{1-8}$ alkyl,
(i) hydroxyl, or
(j) halogen,
with the proviso that heterocycle is not phthalimide;
comprising the step of thiolating

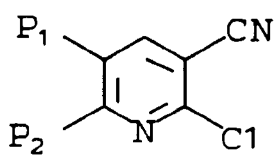

to yield

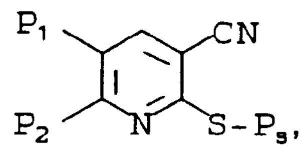

wherein $P_s$ is a thio protecting group;
in a process yielding a compound of formula V.

European Patent Office

# EUROPEAN SEARCH REPORT

Application Number

EP 91 30 9618
PAGE1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | CHEMICAL ABSTRACTS, vol. 115, no. 13, 30 September 1991, Columbus, Ohio, US; abstract no. 126401T, W.S. SAARI ET AL.: '2-Pyridinone derivatives: a new class of nonnucleoside, HIV-1-specific reverse transcriptase inhibitors' page 24 ; & J. Med. Chem. 1991, 34(9), 2922-5 | 1-3,5-9, 23-26 | C07D413/12<br>C07D413/06<br>C07D213/64<br>C07D213/70<br>C07D213/73<br>A61K31/44<br>//(C07D413/12, 263:00,213:00)<br>(C07D413/06, 263:00,213:00) |
| A | US-A-4 461 901 (J.R. POTOSKI ET AL.) * column 3, lines 10-15, chemical formula * | 1 | |
| A | CHEMICAL ABSTRACTS, vol. 94, no. 5, 2 February 1981, Columbus, Ohio, US; abstract no. 30805Z, page 580 ; & JP-A-80-83786 (DAINIPPON PHARMACEUTICAL CO., LTD.), 24-06-1980 | 1 | |
| A | CHEMICAL ABSTRACTS, vol. 99, no. 17, 24 October 1983, Columbus, Ohio, US; abstract no. 139717B, M.A. HASSAN ET AL.: 'Synthesis and reactions of polysubstituted 2(1H)-pyridones and pyridines' page 579 ; & Pol. J. Chem. 1982, 56(2), 419-23 | 1 | **TECHNICAL FIELDS SEARCHED (Int. Cl.5)**<br>C07D<br>C07H<br>A61K |
| A,D | US-A-3 721 676 (B.E. WITZEL ET AL.) | 1 | |
| A | WO-A-8 907 939 (UPJOHN CO.) * page 5, lines 29-34; page 8; claim 1 * | 23-26 | |
| A | EP-A-0 308 977 (ZAIDAN HOJIN BISEIBUTSU KAGAKU KENKYU KAI) * abstract; claims 1,15,16 * | 23-26 | |
| | -/-- | | |

The present search report has been drawn up for all claims

| Place of search | Date of completion of the search | Examiner |
|---|---|---|
| BERLIN | 31 JANUARY 1992 | HASS C. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number

EP 91 30 9618
PAGE2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A,D | JOURNAL OF HETEROCYCLIC CHEMISTRY<br>vol. 20, 1983,<br>pages 199 - 203;<br>C.O. OKAFOR ET AL.: 'Synthesis of Analogues of the 2,3,6-Triazaphenothiazine Ring System (1)'<br>* page 199, scheme 1, compound 9 *<br>--- | 27,28 | |
| A,D | JOURNAL OF ORGANIC CHEMISTRY.<br>vol. 47, 1982, EASTON US<br>pages 592 - 594;<br>C.O. OKAFOR: 'Studies in the Heterocyclic Series. 22. New Chemistry of Azaphenothiazine and Its Precursors'<br>* page 592, right column, compound 3 *<br>----- | 31 | |
| | | | TECHNICAL FIELDS SEARCHED (Int. Cl.5 ) |

The present search report has been drawn up for all claims

| Place of search | Date of completion of the search | Examiner |
|---|---|---|
| BERLIN | 31 JANUARY 1992 | HASS C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)